(19) Europäisches Patentamt
European Patent Office
Office européen des brevets

(11) Publication number: 0 272 868 A1

(12) **EUROPEAN PATENT APPLICATION**

(21) Application number: 87311033.2

(22) Date of filing: 15.12.87

(51) Int. Cl.[4]: C 07 D 487/04
A 61 K 31/55
//(C07D487/04,257:00,243:00),
(C07D487/04,253:00,243:00),
(C07D487/04,251:00,243:00),
(C07D487/04,243:00,239:00)

(30) Priority: 23.12.86 US 945929

(43) Date of publication of application:
29.06.88 Bulletin 88/26

(84) Designated Contracting States:
CH DE FR GB IT LI NL

(71) Applicant: MERCK & CO. INC.
126, East Lincoln Avenue P.O. Box 2000
Rahway New Jersey 07065-0900 (US)

(72) Inventor: Freidinger, Roger M.
2185 Rebecca Drive
Hatfield Pennsylvania 19440 (US)

Evans, Ben E.
501 Perkiomen Avenue
Lansdale Pennsylvania 19446 (US)

Bock, Mark G.
1603 Leon Drive
Hatfield Pennsylvania 19440 (US)

(74) Representative: Hesketh, Alan, Dr.
European Patent Department Merck & Co., Inc. Terlings
Park Eastwick Road
Harlow Essex, CM20 2QR (GB)

(54) **1,4-Benzodiazepines with 6-membered heterocyclic rings.**

(57) Aromatic 1,4-benzodiazepines with fused 6-membered heterocyclic rings which are antagonists of cholecystokinins and/or gastrin, and are useful in the treatment or prevention of CCK-related and/or gastrin-related disorders of the gastrointestinal, central nervous and appetite regulatory systems; composition comprising these compounds; and methods of treatment of mammals or of increasing food intake of animals employing these compounds.

EP 0 272 868 A1

## Description

### 1,4-BENZODIAZEPINES WITH 6-MEMBERED HETEROCYCLIC RINGS

BACKGROUND OF THE INVENTION

Cholecystokinins (CCK) and gastrin are structurally-related neuropeptides which exist in gastrointestinal tissue and in the the central nervous system (see, V. Mutt, Gastrointestinal Hormones, G. B. J. Glass, Ed., Raven Press, N.Y., p. 169 and G. Nisson, ibid, p. 127).

Cholecystokinins include CCK-33, a neuropeptide of thirty-three amino acids in its originally isolated form (see, Mutt and Jorpes, Biochem. J. 125, 678 (1971)), its carboxylterminal octapeptide, CCK-8 (a naturally-occurring neuropeptide, also, and the minimum fully active sequence), and 39- and 12-amino acid forms, while gastrin occurs in 34-, 17- and 14-amino acid forms, with the minimum active sequence being the C-terminal pentapeptide, Gly-Trp-Met-Asp-Phe-$NH_2$, which is the common structural element shared by both CCK and gastrin.

CCK's are believed to be physiological satiety hormones, thereby possibly playing an important role in appetite regulation (G. P. Smith, Eating and Its Disorders, A. J. Stunkard and E. Stellar, Eds, Raven Press, New York, 1984, p. 67), as well as also stimulating colonic motility, gall bladder contraction, pancreatic enzyme secretion, and inhibiting gastric emptying. They reportedly co-exist with dopamine in certain mid-brain neurons and thus may also play a role in the functioning of dopaminergic systems in the brain, in addition to serving as neurotransmitters in their own right (see: A. J. Prange et al., "Peptides in the Central Nervous System", Ann. Repts. Med. Chem. 17, 31, 33 [1982] and references cited therein; J. A. Williams, Biomed. Res. 3 107 [1982]); and J. E. Morley, Life Sci. 30, 479, [1982]).

The primary role of gastrin, on the other hand, appears to be stimulation of the secretion of water and electrolytes from the stomach, and, as such, it is involved in control of gastric acid and pepsin secretion. Other physiological effects of gastrin then include increased mucosal blood flow and increased antral motility, with rat studies having shown that gastrin has a positive trophic effect on the gastric mucosa, as evidenced by increased DNA, RNA and protein synthesis.

Antagonists to CCK and to gastrin have been useful for preventing and treating CCK-related and/or 50strin-related disorders of the gastrointestinal (GI) and central nervous (CNS) systems of animals, especially of humans. Just as there is some overlap in the biological activities of CCK and gastrin, antagonists also tend to have affinity for both receptors. In a practical sense, however, there is enough selectivity to the different receptors that greater activity against specific CCK- or gastrin-related disorders can often also be identified.

Selective CCK antagonists are themselves useful in treating CCK-related disorders of the appetite regulatory systems of animals as well as in potentiating and prolonging opiate-mediated analgesia, thus having utility in the treatment of pain [see P. L. Faris et al., Science 226, 1215 (1984)], while selective gastrin antagonists are useful in the modulation of CNS behavior, as a palliative for gastrointestinal neoplasms, and in the treatment and prevention of gastrin-related disorders of the gastrointestinal system in humans and animals, such as peptic ulcers, Zollinger-Ellison syndrome, antral G cell hyperplasia and other conditions in which reduced gastrin activity is of therapeutic value.

Also, since CCK and gastrin also have trophic effects on certain tumors [K. Okyama, Hokkaido J. Med. Sci., 60, 206-216 (1985)], antagonists of CCK and gastrin are useful in treating these tumors [see, R.D. Beauchamp et al., Ann. Surg., 202,303 (1985)].

Four distinct chemical classes of CCK-receptor antagonists have been reported. The first class comprises derivatives of cyclic nucleotides, of which dibutyryl cyclic GMP has been shown to be the most potent by detailed structure-function studies (see, N. Barlos et al., Am. J. Physiol., 242, G 161 (1982) and P. Robberecht et al., Mol., Pharmacol., 17, 268 (1980)).

The second class comprises peptide antagonists which are C-terminal fragments and analogs of CCK, of which both shorter (Boc-Met-Asp-Phe-$NH_2$, Met-Asp-Phe-$NH_2$), and longer (Cbz-Tyr($SO_3H$)-Met-Gly-Trp-Met-Asp-$NH_2$) C-terminal fragments of CCK can function as CCK antagonists, according to recent structure-function studies (see, R. T. Jensen et al., Biochem. Biophys. Acta., 757, 250 (1983), and M. Spanarkel et al., J. Biol. Chem., 258, 6746 (1983)). The latter compound was recently reported to be a partial agonist [see, J. M. Howard et al., Gastroenterology 86(5) Part 2, 1118 (1984)].

Then, the third class of CCK-receptor antagonists comprises the amino acid derivatives: proglumide, a derivative of glutaramic acid, and the N-acyl tryptophans including para-chlorobenzoyl-L-tryptophan (benzotript), [see, W. F. Hahne et al., Proc. Natl. Acad. Sci. U.S.A., 78, 6304 (1981), R. T. Jensen et al., Biochem. Biophys. Acta., 761, 269 (1983)]. All of these compounds, however, are relatively weak antagonists of CCK ($IC_{50}$: generally $10^{-4}$M[although more potent analogs of proglumide have been recently reported in F. Makovec et al., Arzneim-Forsch Drug Res., 35 (II), 1048 (1985) and in German Patent Application DE 3522506A1], but down to $10^{-6}$M in the case of peptides), and the peptide CCK-antagonists have substantial stability and absorption problems.

In addition, a fourth class consists of improved CCK-antagonists comprising a nonpeptide of novel structure from fermentation sources [R. S. L. Chang et al., Science, 230, 177-179 (1985)] and 3-substituted benzodiazepines based on this structure [published European Patent Applications 167 919, 167 920 and 169 392, B. E. Evans et al, Proc. Natl. Acad. Sci. U.S.A., 83, p. 4918-4922 (1986) and R.S.L. Chang et al, ibid,

p. 4923-4926] have also been reported.

No really effective receptor antagonists of the in vivo effects of gastrin have been reported (J. S. Morley, Gut Pept. Ulcer Proc., Hiroshima Symp. 2nd, 1983, p. 1), and very weak in vitro antagonists, such as proglumide and certain peptides have been described [(J. Martinez, J. Med. Chem. 27, 1597 (1984)]. Recently, however, pseudopeptide analogs of tetragastrin have been reported to be more effective gastrin antagonists than previous agents [J. Martinez et al., J. Med. Chem., 28, 1874-1879 (1985)].

The benzodiazepine (BZD) structure class, which has been widely exploited as therapeutic agents, especially as central nervous system (CNS) drugs, such as anxiolytics, and which exhibits strong binding to "benzodiazepine receptors" in vitro, has not in the past been reported to bind to CCK or gastrin receptors. Benzodiazepines have been shown to antagonize CCK-induced activation of rat hippocampal neurones but this effect is mediated by the benzodiazepine receptor, not the CCK receptor [see J. Bradwejn et al., Nature, 312, 363 (1984). Of these reported BZD's, additionally, the large majority do not contain substituents attached to the 3-position of the seven membered ring, as it is well known in the art that 3-substituents result in decreasing anxiolitic activity, especially as these substituents increase in size. Further, it has been demonstrated that in the case of the 3-substituted benzodiazepines that have been reported, the preferred stereochemistry at position 3 for CNS activity is S, which would correspond to an L-amino acid, such as L-tryptophan.

It was, therefore, an object of this invention to identify substances which more effectively antagonize the function of cholecystokinins and/or gastrin in disease states in mammals, especially in humans. It was another object of this invention to prepare novel compounds which more selectively inhibit cholecystokinins or inhibit gastrin. It was still another object of this invention to develop a method of antagonizing the functions of cholecystokinin and/or gastrin in disease states in mammals. It is also an object of this invention to develop a method of preventing or treating disorders of the gastrointestinal, central nervous and appetite regulatory systems of mammals, especially of humans, or of increasing food intake of animals.

## SUMMARY OF THE INVENTION

The present invention is directed to aromatic 1,4-benzodiazepines with 6-membered heterocyclic rings of Formula I are antagonists of cholecystokinins (CCK) and/or gastrin, and are useful in the treatment and prevention of CCK-related and/or gastrin-related disorders of the gastrointestinal, central nervous and appetite regulatory systems of mammals, especially of humans, including irritable bowel syndrome, ulcers, excess pancreatic or gastric secretion, acute pancreatitis, motility disorders, neuroleptic disorders, tardive dyskinesia, Parkinson's disease, psychosis, Gilles de la Tourette Syndrome, disorders of the appetite regulatory system, Zollinger-Ellison syndrome, antral G cell hyperplasia, pain (by the potentiation of opioid analgesics), and malignancies of the lower esophagus, stomach, intestines, and colon. As antagonists of CCK, they may also be used to increase food intake in animals.

## DETAILED DESCRIPTION OF THE INVENTION

The compounds of this invention are those of Formula I:

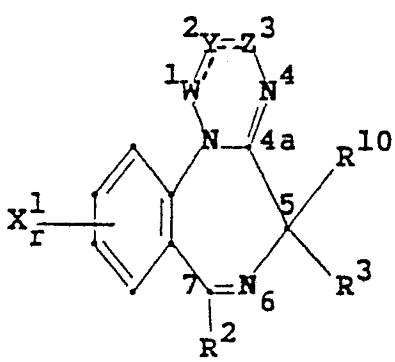

(I)

wherein

$R^1$ is H, $C_1$-$C_4$-alkyl, cyclo-$C_3$-$C_7$-alkyl,

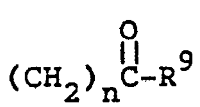 or 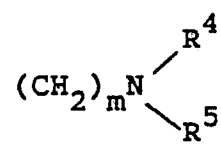 ;

$R^2$ is H, $C_1$-$C_4$-alkyl, mono- or disubstituted or unsubstituted phenyl (where the substituent(s)is/are

independently selected from the group consisting of halo, $C_1$-$C_4$-alkyl, $C_1$-$C_4$-alkoxy, $C_1$-$C_4$-alkylthio, carboxyl, carboxy-$C_1$-$C_4$-alkyl, nitro, $-CF_3$, $O\overset{O}{\overset{\|}{C}}-R^4$ and hydroxy), 2-, 3-, 4-pyridyl, or $(CH_2)_mCOOR^6$;

$R^3$ is $-(CH_2)_nR^7$, $-(CH_2)_n\overset{OH}{\overset{|}{C}}HR^7$, $-(CH_2)_n\overset{O}{\overset{\|}{C}}R^7$,

$-(CH_2)_nNR^{18}(CH_2)_qR^7$, $-(CH_2)_nX^9\overset{O}{\overset{\|}{C}}(CH_2)_qR^7$,

$-(CH_2)_nX^9\overset{O}{\overset{\|}{C}}\underset{NHCOOR^{14}}{\underset{|}{C}H}CH_2R^7$,

$-(CH_2)_nX^9\overset{O}{\overset{\|}{C}}(CH_2)_qX^9_a-$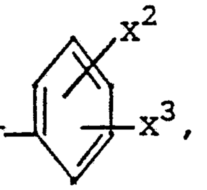,

$-(CH_2)_n-X^9-\overset{O}{\overset{\|}{C}}-X^9_a-(CH_2)_q-R^7$, or

$-(CH_2)_n-X^9-\overset{O}{\overset{\|}{C}}-\overset{NH_2}{\overset{|}{C}H}-CH_2R^7$;

$R^4$ and $R^5$ are independently H, $C_1$-$C_4$-alkyl, or cyclo-$C_3$-$C_7$-alkyl, or are connected to form a hetero ring of the structure $-\overset{\frown}{N(CH_2)_n}$, wherein n is 2 to 6;

$R^6$ is H, $C_1$-$C_4$-alkyl, cyclo-$C_3$-$C_7$-alkyl, unsubstituted or mono- or disubstituted phenyl (where the substituents are selected from the group consisting of halo, $C_1$-$C_4$-alkyl, $C_1$-$C_4$-alkoxy, nitro, and $CF_3$), or unsubstituted or mono- or disubstituted phenyl-$C_1$-$C_4$-alkyl (where the substituents are selected from the group consisting of halo, $C_1$-$C_4$-alkyl, $C_1$-$C_4$-alkoxy, nitro, and $CF_3$);

$R^7$ is α- or β-naphthyl, unsubstituted or mono- or disubstituted phenyl (where the substituents are selected from the group consisting of halo, $-NO_2$, -OH, $-NR^4R^5$, $C_1$-$C_4$-alkyl, cyano, phenyl, trifluoromethyl, acetylamino, acetyloxy, $C_1$-$C_4$-alkylthio, $SCF_3$, $C\equiv CH$, $CH_2SCF_3$, S-phenyl, or $C_1$-$C_4$-alkoxy);

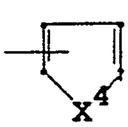 , 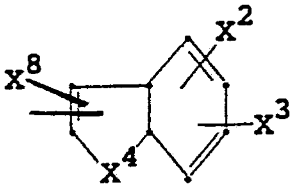 , 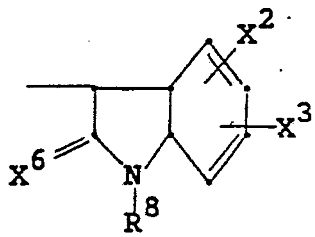

-CH=CH 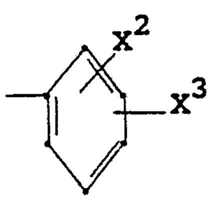 , or -CH=CH 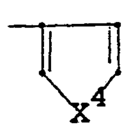 ,

$R^8$ is H, $C_1$-$C_4$-alkyl, cyclo-$C_3$-$C_7$-alkyl, $-(CH_2)_n$-cyclo-$C_3$-$C_7$-alkyl, $-\overset{O}{\overset{\|}{C}}-C_1$-$C_4$-alkyl, or

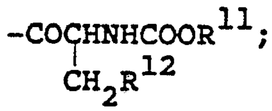

$R^9$ is OH, $OR^{11}$ or

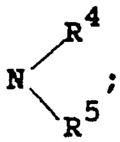

$R^{10}$ is H, -OH, or $-CH_3$;
$R^{11}$ and $R^{12}$ are independently $C_1$-$C_4$-alkyl or cyclo-$C_3$-$C_7$-alkyl;
$R^{14}$ is $C_1$-$C_4$-alkyl or phenyl-$C_1$-$C_4$-alkyl;
$R^{18}$ is H, $C_1$-$C_4$-alkyl, formyl, acetyl, propionyl or butyryl;
m is 1 to 4;
n is 0 to 4;
q is 0 to 4;
r is 1 to 2;
$X^1$ is H, $-NO_2$, $CF_3$, CN, OH, $C_1$-$C_4$-alkyl, halo, $C_1$-$C_4$-alkylthio, $C_1$-$C_4$-alkoxy, $-(CH_2)_nCOOR^6$, $-NR^4R^5$, or $O-\overset{O}{\overset{\|}{C}}-R^4$;
$X^2$ and $X^3$ are independently H, -OH, $-NO_2$, halo, $C_1$-$C_4$-alkylthio, $C_1$-$C_4$-alkyl, $C_1$-$C_4$-alkoxy or $-O-\overset{O}{\overset{\|}{C}}-R^4$;
$X^4$ is S, O, $CH_2$, or $NR^8$;
$X^6$ is O or HH;
$X^8$ is H or $C_1$-$C_4$-alkyl;
$X^9$ and $X^9_a$ are independently $NR^{18}$ or O;
W is $CR^1$, $CHR^{10}$ or $\overset{O}{\overset{\|}{C}}$;
Y is $N-R^1$, $C-R^1$, or $\overset{O}{\overset{\|}{C}}$;
Z is N, $NR^1$, $CR^1$, or $\overset{O}{\overset{\|}{C}}$;
--- is a saturated (single) or unsaturated (double) bond;
and the pharmaceutically-acceptable salts thereof.

The stereochemistry of the compounds may be D, L or DL.

As used herein, the definition of each expression, e.g. m, n, p, R, X, $C_1$-$C_4$-alkyl, etc., when it occurs more than once in any structure, is intended to be independent of its definition elsewhere in the same structure.

However, in the compounds of Formula I, the preferred stereochemistry for CCK-antagonism relates to D-tryptophan, where $C^{4a}$ and $N^6$ of Formula I correspond to the carbonyl carbon and α-amino nitrogen of D-tryptophan respectively, and $R^3$ occupies the position of the indolylmethyl side chain. Then, in the compounds of Formula I, the preferred stereochemistry for gastrin antagonism may be either D or L depending on the nature of $R^3$. For example, when $R^3$ is $(CH_2)_nR^7$ or $(CH_2)_nX^9\overset{O}{\overset{\|}{C}}(CH_2)_q$, the preferred stereochemistry corresponds to D-tryptophan, as above, and when $R^3$ is $(CH_2)_n-X^9-\overset{O}{\overset{\|}{C}}-X^9_a-(CH_2)_n-R^7$, the the preferred stereochemistry corresponds to L-tryptophan.

As used herein, halo is F, Cl, Br, or I; $C_1$-$C_4$-alkyl is straight or branched-chain alkyl and includes methyl, ethyl, propyl, isopropyl, butyl, isobutyl, and t-butyl; and in $C_1$-$C_4$-alkoxy and $C_1$-$C_4$-alkylthio, the alkyl portion is as previously defined.

Preferred compounds according to the present invention are those wherein $R^1$ is H or methyl, $R^2$ is phenyl or o-F-phenyl, $R^3$ is $NH\overset{O}{\overset{\|}{C}}R^7$ or $NH\overset{O}{\overset{\|}{C}}NHR^7$,

R⁷ is 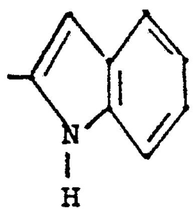 , 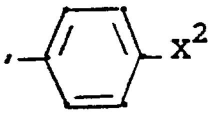 , 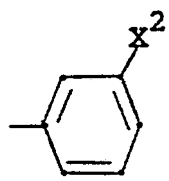 or 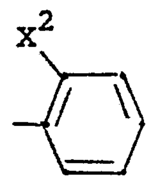 ,

$X^1$ is H and $X^2$ is H, $-NO_2$, halo, methyl, or methoxy, and either:
W is $CR^1$, Y is $CR^1$ and Z is $\overset{O}{\overset{\|}{C}}$; or W is $\overset{O}{\overset{\|}{C}}$, Y is $CR^1$ and Z is $CR^1$;
or W is $\overset{O}{\overset{\|}{C}}$, Y is $CR^1$ and Z is N;
or W is $\overset{O}{\overset{\|}{C}}$, Y is $NR^1$ and Z is $\overset{O}{\overset{\|}{C}}$, or W is $CHR^1$, Y is $\overset{O}{\overset{\|}{C}}$, and Z is $NR^1$. Particularly, for preventing gastrin-related problems, preferred compounds are those wherein
$R^3$ is $NH\overset{O}{\overset{\|}{C}}NHR^7$,

$R^7$ is 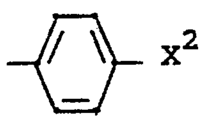 or 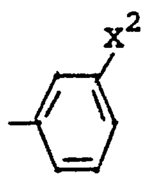 ,

and the stereochemistry corresponds to L-tryptophan. For preventing and treating CCK-related problems, preferred compounds include those wherein
$R^3$ is $NH\overset{O}{\overset{\|}{C}}R^7$,

$R^7$ is 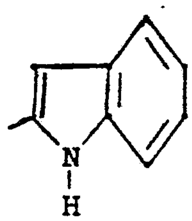 or 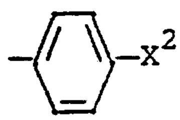 ,

$X^2$ is halo and wherein
$R^3$ is $NH\overset{O}{\overset{\|}{C}}NHR^7$,

$R^7$ is 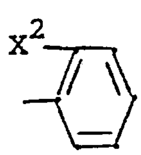 ,

and the stereochemistry corresponds to D-tryptophan.
Such particularly preferred compounds include, for CCK antagonism:
5(S)1,5-dihydro-5-(2-indolecarbonylamino)-2-methyl-7-phenyl-as-triazino[4,2-a][1,4]benzodiazepine;
5(S)-5-(4-chlorophenylcarbonylamino)-3,5-dihydro-3-methyl-7-phenyl-as-triazino-[4,3-a][1,4]benzodiazepine-2(1H)one;
5(S)-5-(2-indolecarbonylamino)-1-methyl-7-phenyl-5H-pyrimido[4,3-a[1,4]-benzodiazepin-3-one;
5(S)-5-(2-indolecarbonylamino)-2-methyl-7-phenyl-5H-pyrimido-[4,3-a][1,4]-benzodiazepin-1-one; and
5(S)-5-(2-indolecarbonylamino)-2-methyl-7-phenyl-s-triazino-[4,3-a][1,4]-benzodiazepin-1,3-dione;

and for gastrin antagonism:
5(R)1,5-dihydro-5-(3-methoxyphenylaminocarbonylamino)-2-methyl-7-phenyl-as-triazino[4,3-a][1,4]benzodiazepine;
5(R)3,5-dihydro-3-methyl-5-(3-methylphenylaminocarbonylamino)-7-phenyl-as-triazino[4,3-a][1,4]-benzodiazepine-2(1H)one;
5(R)-5-(3-chlorophenylaminocarbonylamino)1-methyl-7-phenyl-5H-pyrimido-[4,3-a][1,4]-benzodiazepin-3-one;
5(R)-5-(3-methoxyphenylaminocarbonylamino)-2-methyl-7-phenyl-5H-pyrimido-[4,3-a][1,4]-benzodiazepine-1-one; and
5(R)-5-(3-methoxyphenylaminocarbonylamino)-2-methyl-7-phenyl-s-triazino-[4,3-a][1,4]-benzodiazepin-1,3-dione.

The pharmaceutically-acceptable salts of the compounds of Formula I include the conventional non-toxic salts or the quarternary ammonium salts of the compounds of Formula I formed, e.g., from non-toxic inorganic or organic acids. For example, such conventional non-toxic salts include those derived from inorganic acids such as hydrochloric, hydrobromic, sulfuric, phosphoric, nitric and the like; and the salts prepared from organic acids such as acetic, propionic, succinic, glycolic, stearic, lactic, malic, tartaric, citric, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, isethionic, and the like.

The compounds of Formula I are particularly distinguished from benzodiazepines of the prior art by the presence of 3-substituents. These Formula I compounds bind strongly to CCK-receptors, but only weakly to benzodiazepine-receptors, especially with the increasing size of the 3-substituent.

Compounds according to Formula (I) may be prepared according to Scheme I through VIII as follows:

SCHEME I

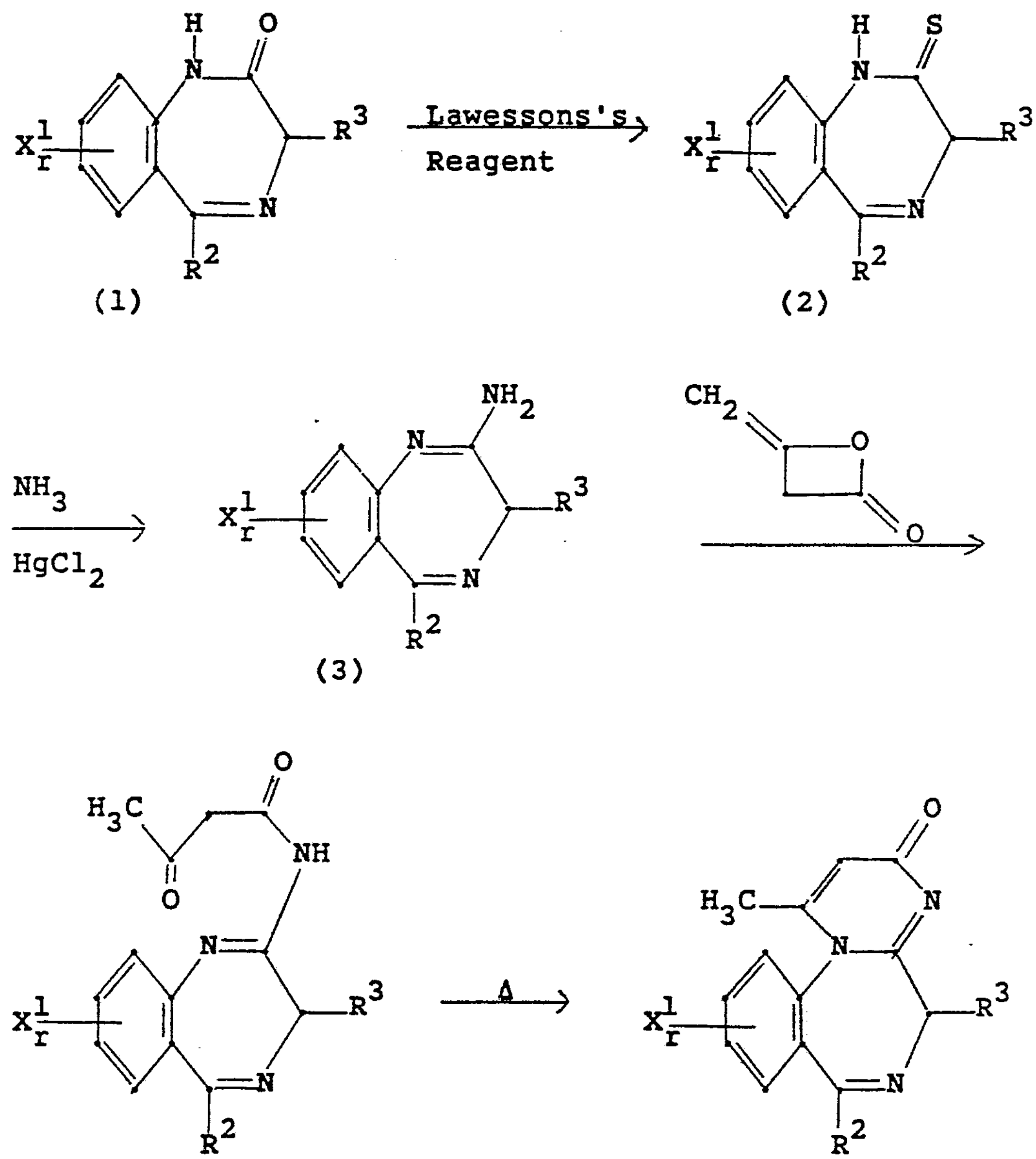


(1) (2) (3) (4) (5)

SCHEME II

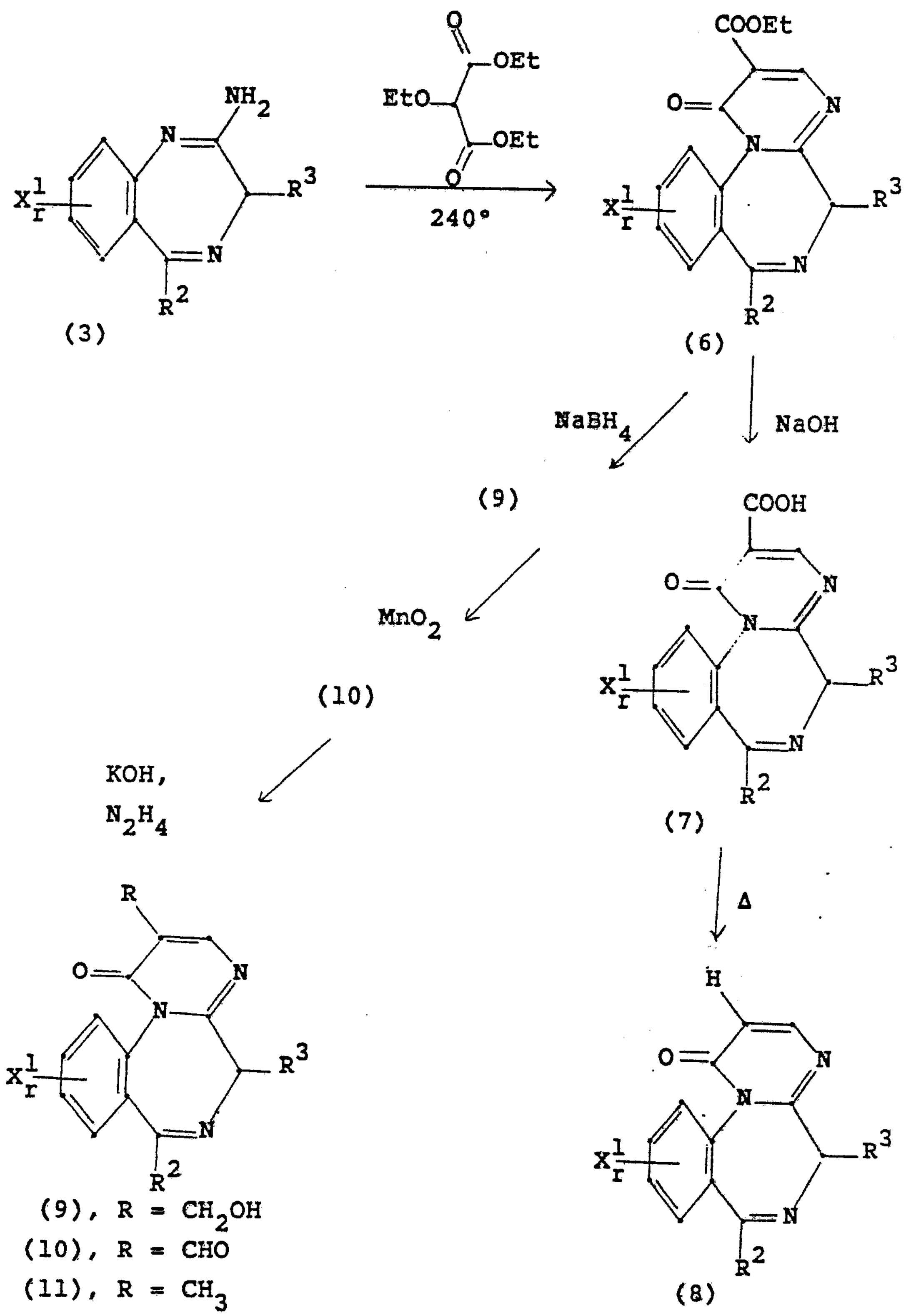


(3) (6) (9) (7) (10) (8)

(9), R = $CH_2OH$
(10), R = CHO
(11), R = $CH_3$

SCHEME III

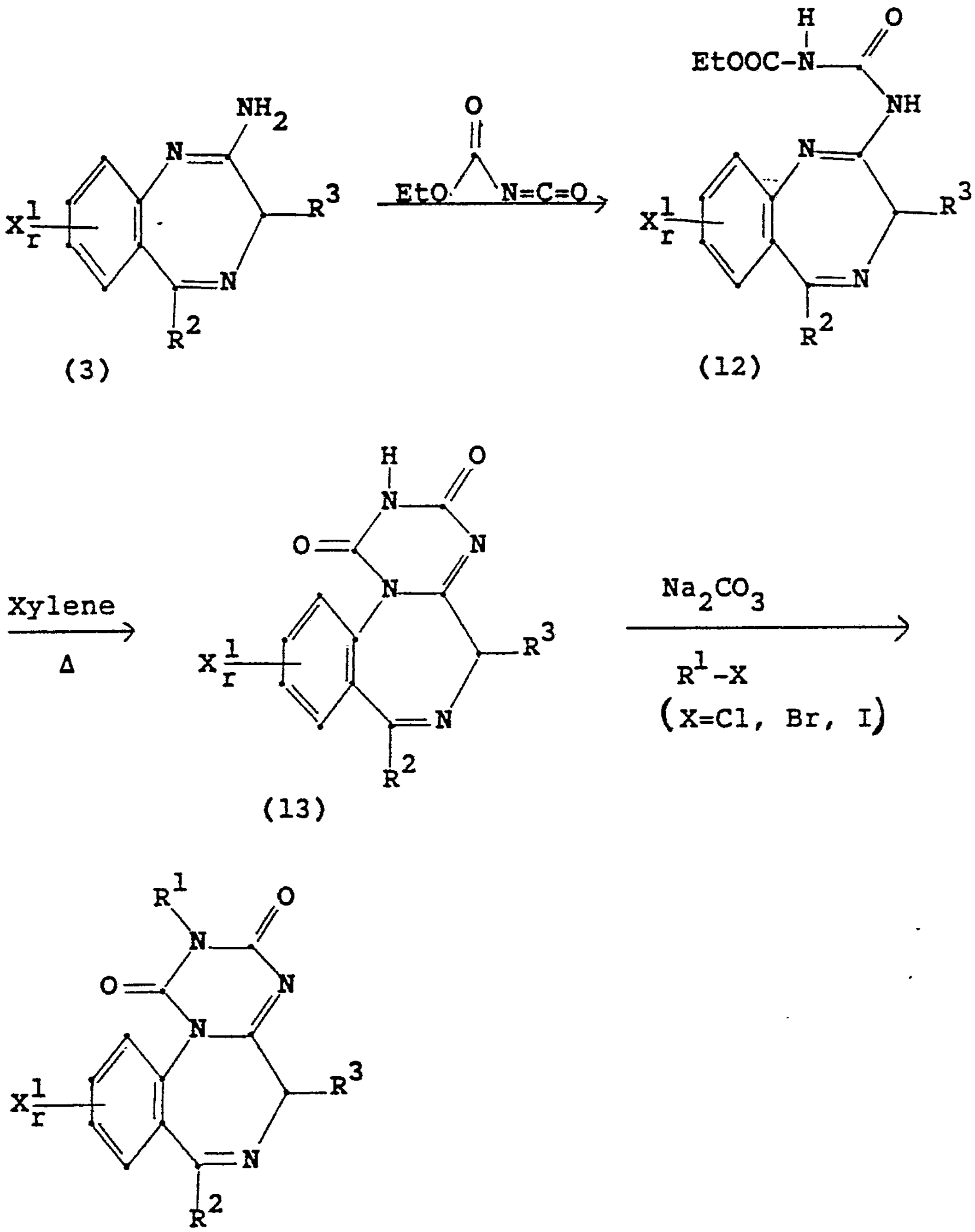


(3) (12) (13) (14)

SCHEME IV

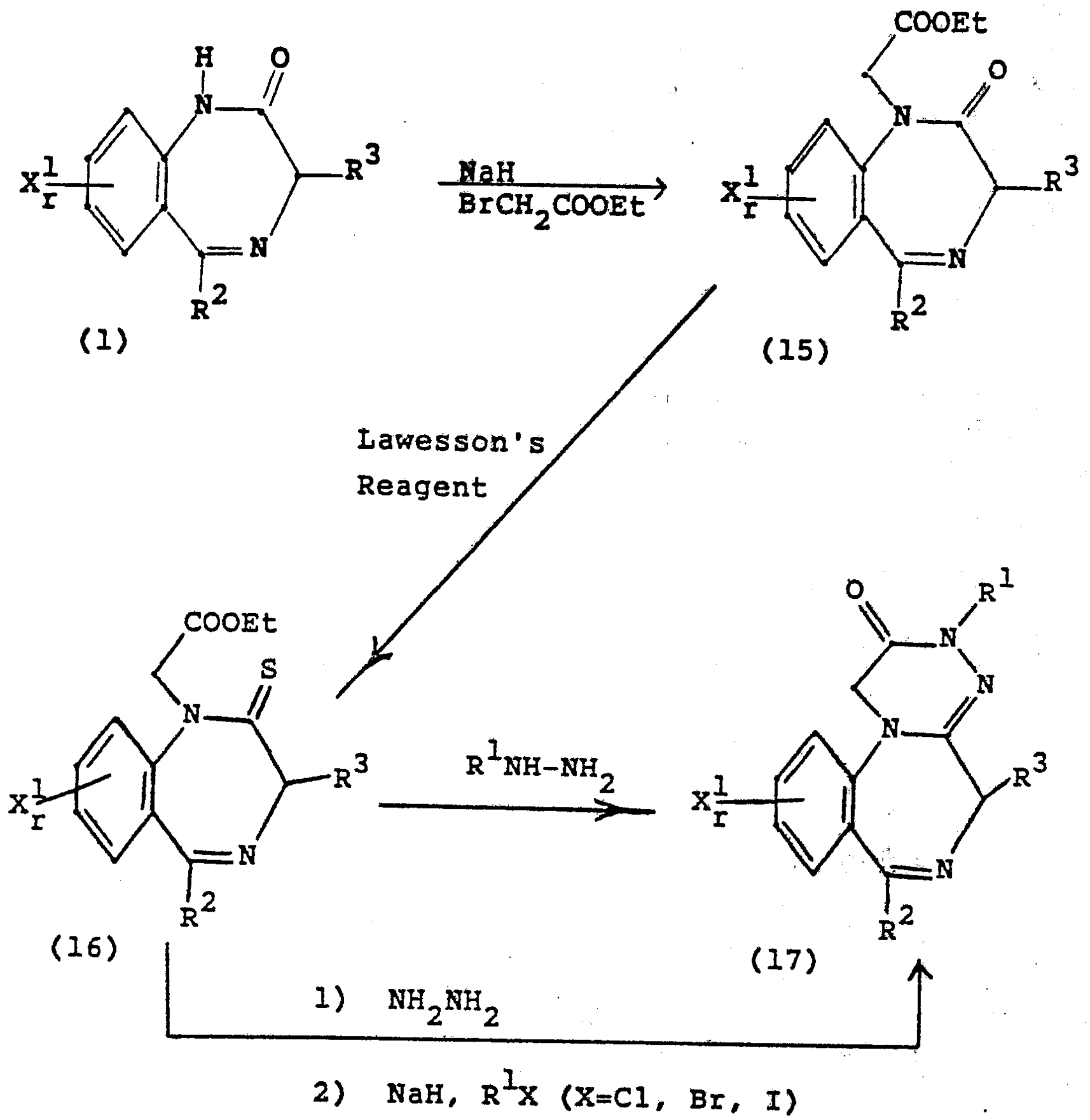
NaH
BrCH2COOEt
(1)
(15)
Lawesson's Reagent
(16)
(17)
R1NH-NH2
1) NH2NH2
2) NaH, R1X (X=Cl, Br, I)

SCHEME V

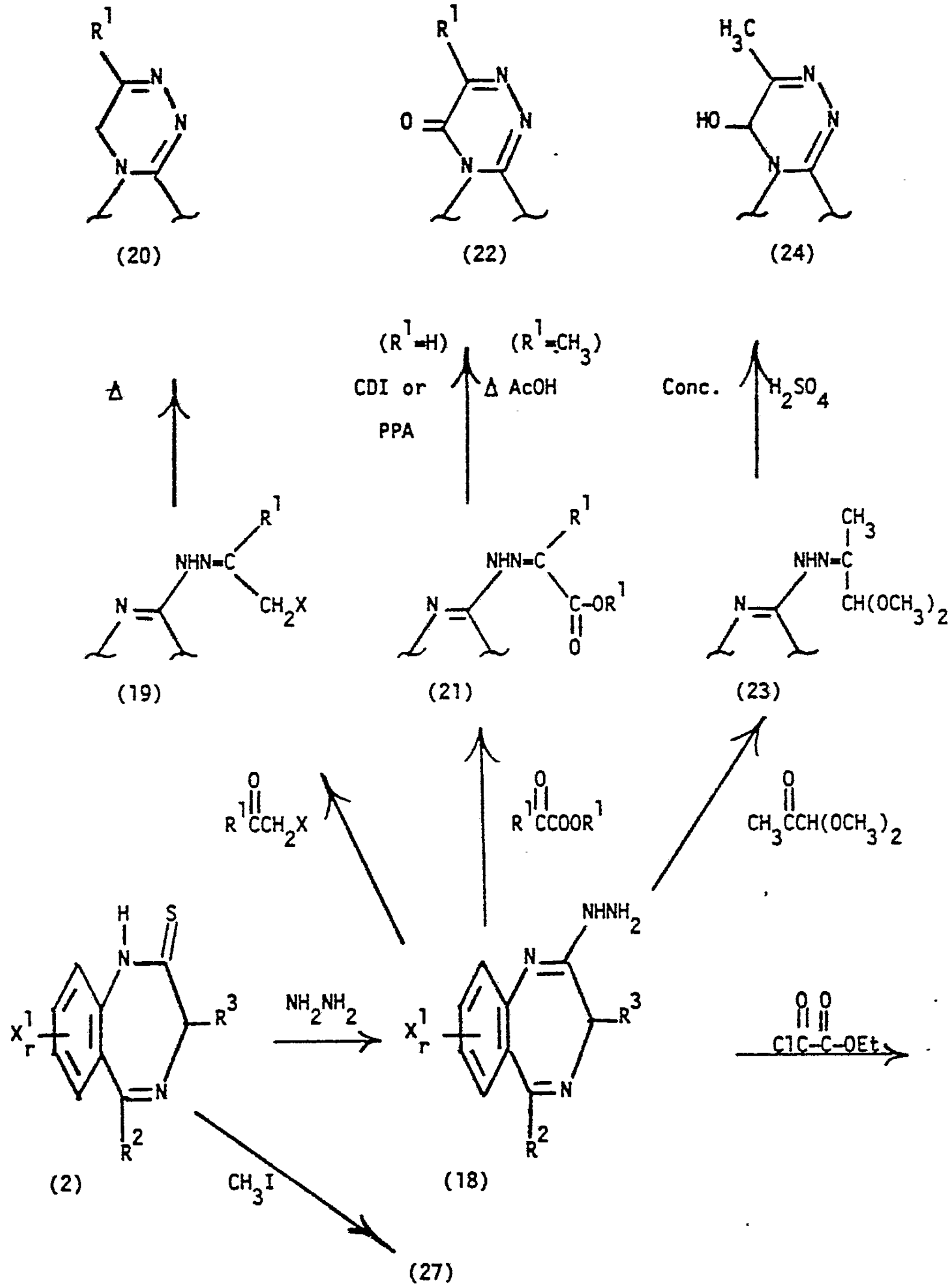
(20)
(22)
(24)
Δ
(R¹=H)
(R¹=CH₃)
CDI or
PPA
Δ AcOH
Conc.
H₂SO₄
(19)
(21)
(23)
NH₂NH₂
CH₃I
(2)
(18)
(27)

SCHEME V (Cont'd):

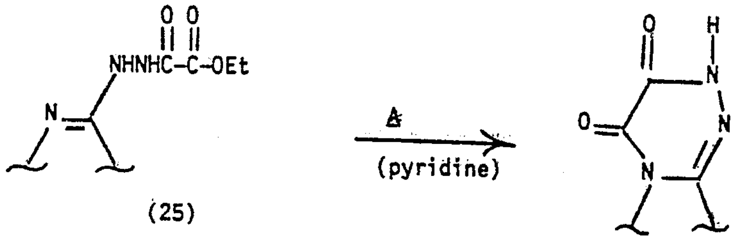


(25)

(26)

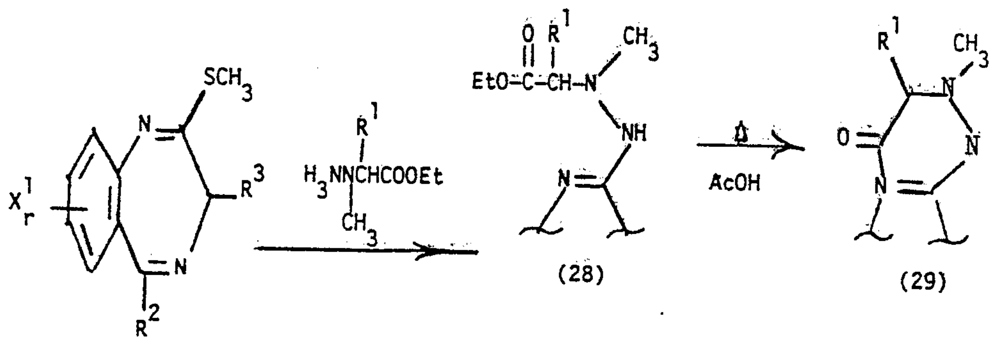


(28)

(29)

(27)

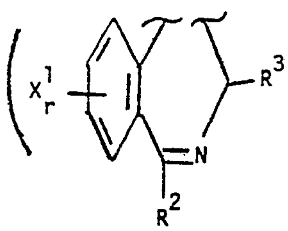

Base structure for (19)-(26) and (28)-(29).

REACTION SCHEME VI

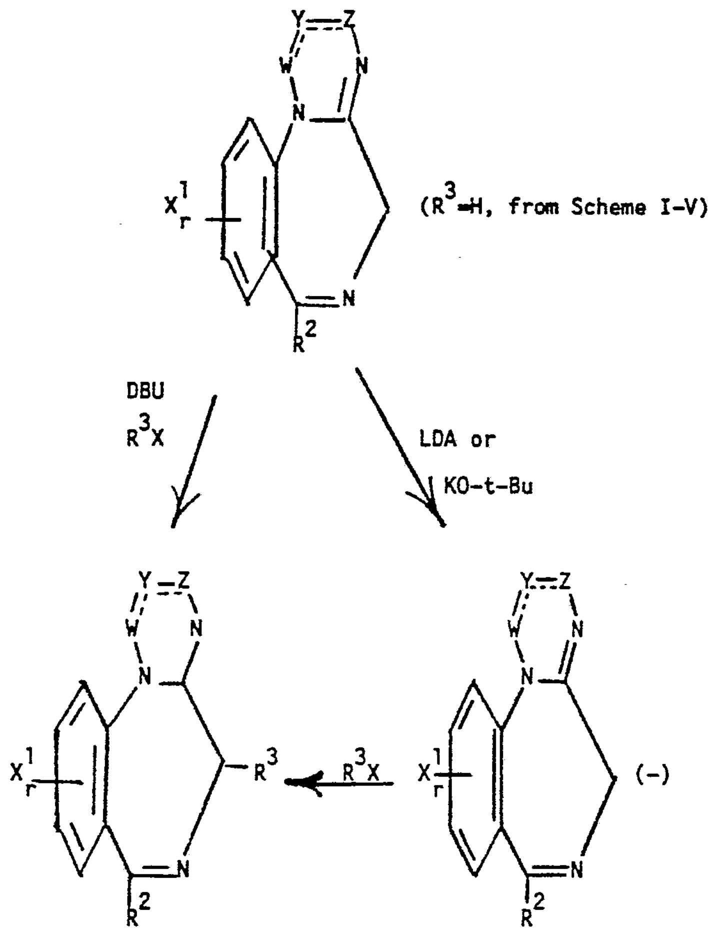


(30) (31)

REACTION SCHEME VI (cont'd)

(31)

(31)

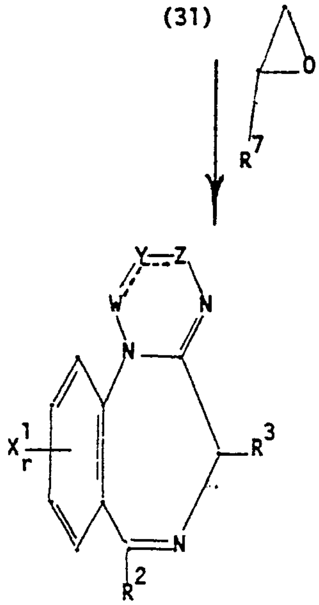

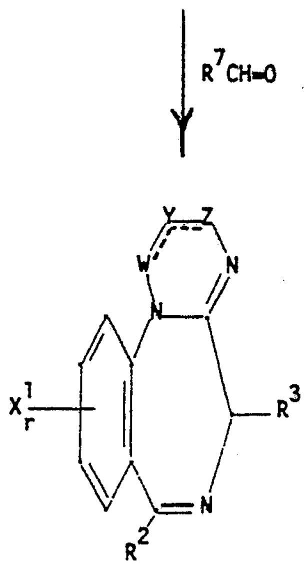


(32)

(33)

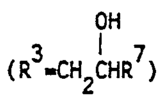

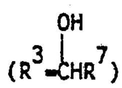

(n is at least 1 where the attachment atom to $R^7$ is C; otherwise n is at least 2)

REACTION SCHEME VII

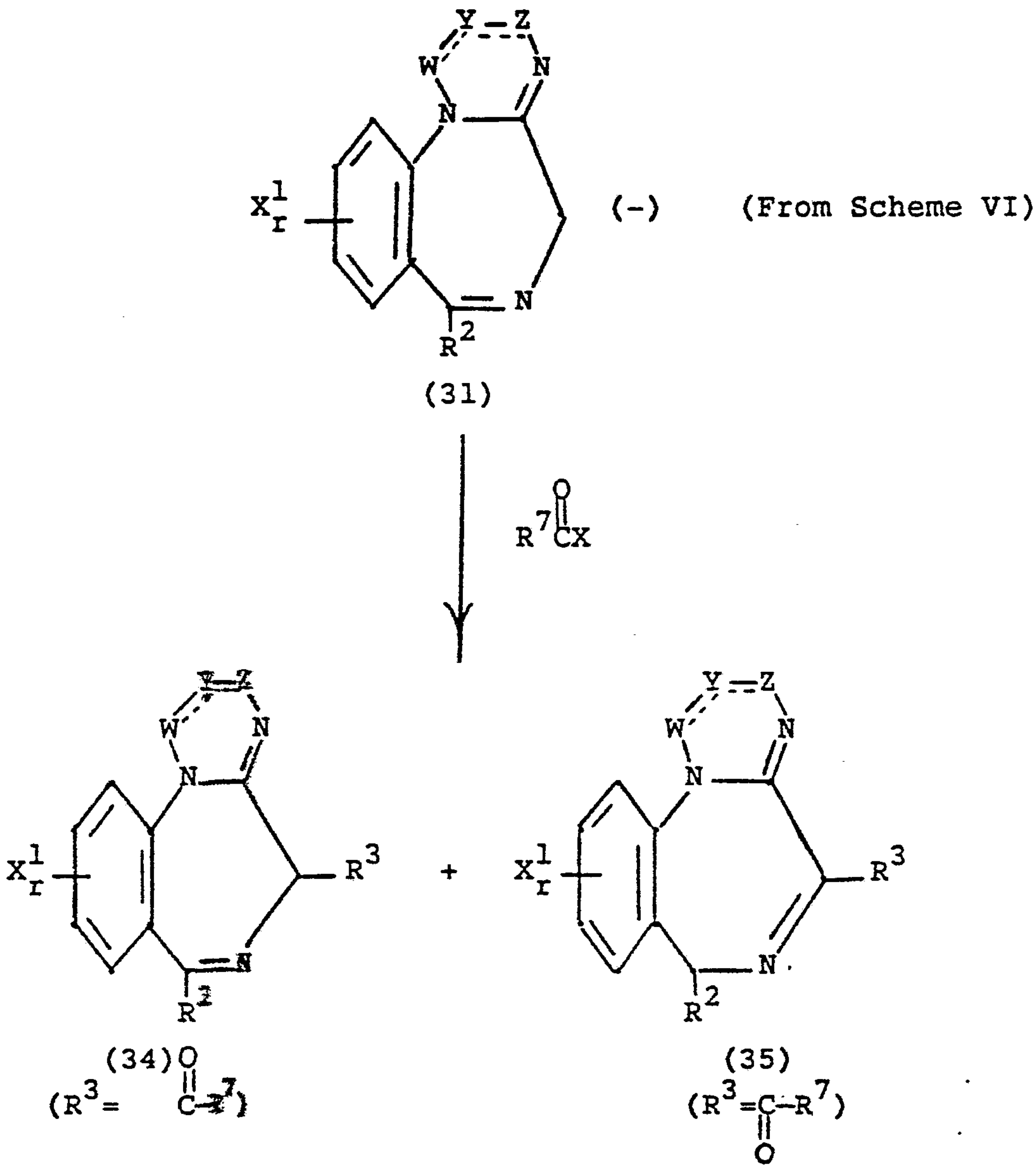


(31)

(34)

(35)

REACTION SCHEME VII (Cont'd)

(34)

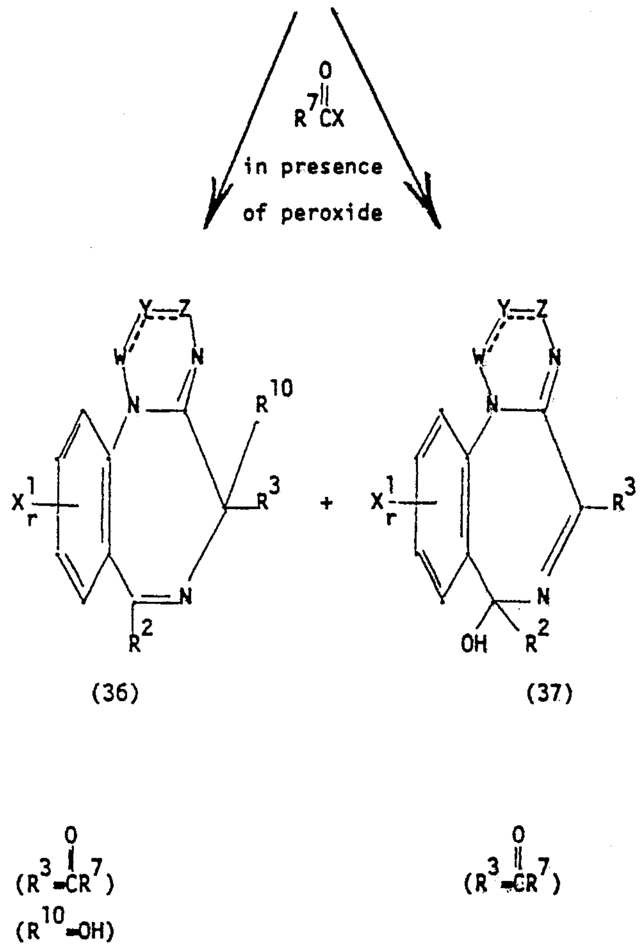


(36)

(37)

REACTION SCHEME VIII

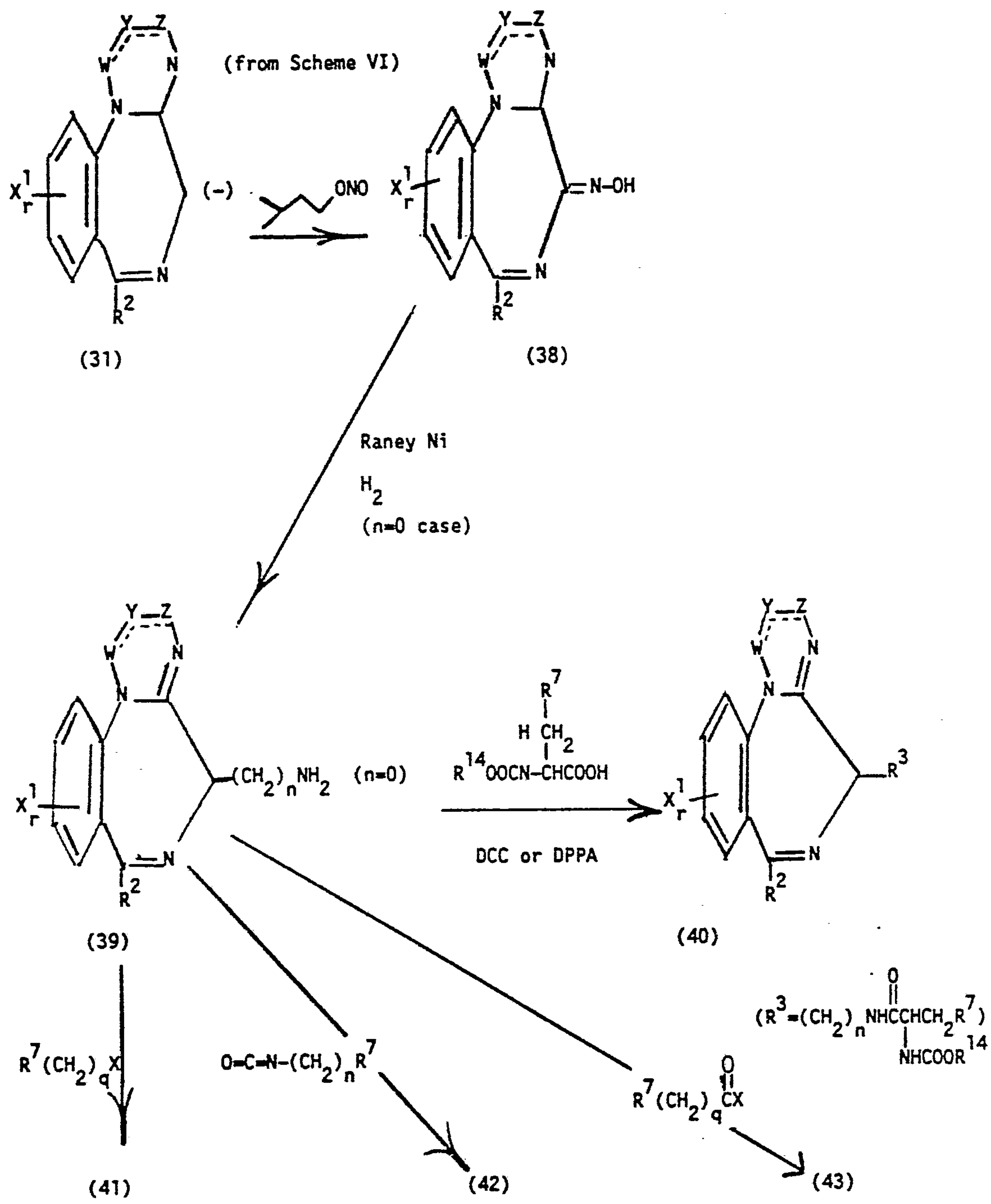

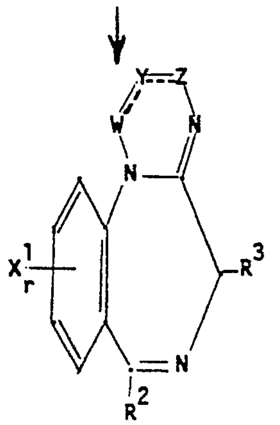

(41)

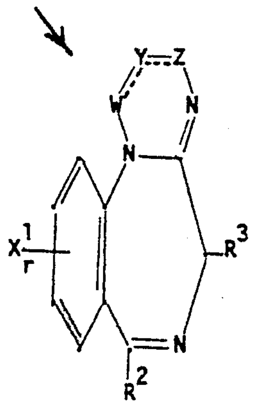

(42)

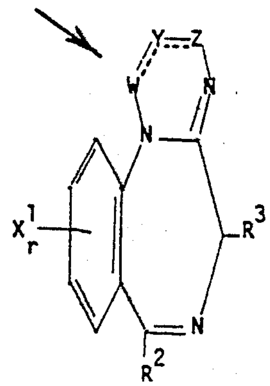

(43)

$R^3 = (CH_2)_{0-1}NH(CH_2)_qR^7$

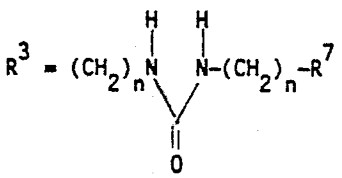

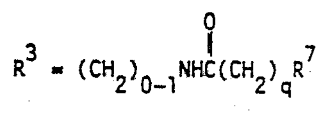

Referring to Reaction Scheme I, the 1,4-benzodiazepine (1), prepared according to methods known in the art, is treated with Lawesson's reagent to produce the thiolactam (2). Compound (2) is treated with ammonia in the presence of mercury II salts such as mercuric chloride to yield amidine (3). Reaction of (3) with diketene gives (4) which upon heating provides pyrimidobenzodiazepin-3-one (5).

Referring now to Reaction Scheme II, amidine (3) from Reaction Scheme I is heated with ethoxymalonic acid diester to give pyrimidobenzodiazepines with a carbonyl group in position 1. Upon saponification, the acid (7) is obtained, and this substance is decarboxylated to (8). Alternatively, reduction of (6) with sodium borohydride yields alcohol (9) which upon oxidation with manganese dioxide provides the aldehyde (10). Wolff-Kishner reaction of (10) yields the 2-methyl compounds (11).

Referring to Reaction Scheme III, amidine (3) from Reaction Scheme I is treated with ethoxycarbonyl isocyanate to give Compound (12). Upon heating, this compound is cyclized to the s-triazinodione (13). Alkylation of (13) with alkyl halides provides the 2-alkyl derivatives (14).

Referring now to Reaction Scheme IV, 1,4-benzodiazepine (1) from Reaction Scheme I is alkylated with ethylbromoacetate in the presence of sodium hydride to yield the N-1 alkylated derivative (15). Treatment of (15) with Lawesson's reagent provides the thiolactam (16). Reaction of (16) with alkyl hydrazines results in cyclization to the as-triazinobenzodiazepines (17). Alternatively, reaction of (16) with hydrazine followed by alkylation also gives (17).

Referring to Reaction Scheme V, thiolactam (2) from Reaction Scheme I is treated with hydrazine to give amidrazone (18). Compound (18) reacts with halocarbonyl compounds to produce hydrazones (19) which upon heating yield as-thiazinobenzodiazepines (20). Alternatively, treatment of (18) with carbonyl esters yields hydrazones (21) which are cyclized to 1-carbonyl-as-triazinobenzodiazepines (22) by heating in acetic acid or by treatment with carbonyl diimidazole or polyphosphoric acid. Treatment of (18) with carbonyl acetals gives hydrazones (23) which upon treatment with concentrated sulfuric acid provide 1-hydroxy-as-triazinobenzodiazepines (24). Treatment of (18) with chlorocarbonylethylformate gives oxalic ester hydrazide (25) which upon heating in pyridine yields 1,2-dicarbonyl-as-triazinobenzodiazepine (26). Alkylation of (2) with methyl iodide provides thioiminoether (27). Treatment of (27) with hydrazinoesters yields amidrazonoesters (28) which upon heating in acetic acid gives 1-carbonyl-as-triazinobenzodiazepines (29).

Referring now to Reaction Scheme VI, the anion (31) is generated from compounds produced in Schemes I-V by the procedure of J. Org. Chem., 46, 3945 (1981) using lithium diisopropylamide (LDA) or using potassium tert-butoxide.

(31) can be variously treated. For example, the hydroxy alkyl derivative (33) is generated by adding an aldehyde to a solution of (31). Treatment of (31) with an epoxide yields the hydroxyethyl derivative (32). By treating (31) with an alkyl halide, the alkyl derivative (30) is produced.

An alternative procedure for obtaining (31) is to treat the compounds from Scheme I-V, wherein $R^3 = H$, with a strong base such as 1,8-diazabicyclo-[5.4.0]undec-7-ene (DBU) and heating.

Reaction Scheme VII describes the formation of $R^3$ = keto compounds of Formula I. These are produced by treating the anion (31) with an acid halide or anhydride. This reaction produces both isomers (34) and (35).

When the reaction is run in the presence of peroxide, the hydroxy compounds (36) and (37) are produced.

Reaction Scheme VIII describes the formation of Formula I compounds where $R^3$ is a substituted amino. The amino compounds may be obtained by nitrosation of (31) followed by reduction of the oxime (38) with Raney nickel and hydrogen.

When (39) is treated with an alkyl halide, the N-alkyl derivative (41) is produced.

Treatment of (39) with an acid halide or anhydride produces the N-acyl derivative (43).

Treatment of compounds (39) with an isocyanate gives the ureas (42).

Compound (39) may also be treated with an N-protected α-amino acid and a coupling reagent such as DCC or DPPA (diphenylphosphorylazide) to give the amides of structure (40).

The pharmaceutically-acceptable salts of the present invention may be synthesized from the compounds of Formula I which contain a basic moiety by conventional chemical methods. Generally, the salts are prepared by reacting the free base with stoichiometric amounts of or with an excess of the desired salt-forming inorganic or organic acid in a suitable solvent or in various combinations of solvents.

Screening of the novel compounds according to the present invention to determine biological activity and obtain an $IC_{50}$ value for them, in order to identify significant CCK-antagonism, may be accomplished using an $^{125}I$-CCK-receptor binding assay and in vitro isolated tissue preparations. In order to identify significant gastrin antagonism, $^{125}I$-gastrin and $^{3}H$-pentagastrin binding assays are used. These various tests involve the following:

CCK receptor binding (pancreas) method

CCK-8, radiolabeled with $^{125}I$-Bolton Hunter reagent (2000 Ci/mmole), is purchased from New England Nuclear (NEN) and receptor binding is performed according to Innis and Snyder (Proc. Natl. Acad. Sci. USA, 77, 6917-6921, 1980), with minor modifications as described in Chang and Lotti (Proc. Natl. Acad. Sci. USA, 83, 4923-926, 1986).

The whole pancreas of a male Sprague-Dawley rat (200-350 g), which has been sacrificed by decapitation, is dissected free of fat tissue and homogenized in 20 volumes of ice-cold 50 nM Tris HCl (pH 7.7 at 25°C) with a Brinkmann Polytron PT-10. The homogenates are centrifuged at 48,000 g for 10 minutes, then the resulting pellets are resuspended in Tris Buffer, centrifuged as above, and resuspended in 200 volumes of binding assay buffer (50 mM Tris HCl, pH 7.7 at 25°C, 5 mM dithiothreitol and 0.1 mM bacitracin).

For the binding assay, 25 µl of buffer (for total binding), or unlabeled CCK-8 sulfate sufficient to give a final concentration of 1 µM of CCK-8 (for nonspecific binding), or the compounds according to the instant invention (for determination of antagonism to $^{125}I$-CCK binding) and 25 µl of $^{125}I$-CCK-8 (30,000-40,000 cpm), are added to 450 µl of the membrane suspensions in duplicate or triplicate test tubes. The reaction mixtures are incubated at 37°C for 30 minutes and then filtered on glass fiber GF/B filters which are then rapidly washed with 3 × 4 ml of ice cold Tris HCl containing 1 mg/ml BSA, and the filters are counted with a Beckman Gamma 5000. For Scatchard analysis to determine the mechanism of inhibition of $^{125}I$-CCK binding by the most potent compounds (Ann. N.Y. Acad. Sci., 51, 660, 1949), $^{125}I$-CCK-8 is progressively diluted with increasing concentrations of CCK-8.

CCK receptor binding (brain) method

$^{125}I$-CCK-8 binding is performed similarly to the method described by Saito et al., (J. Neurochem., 37, 483-490, 1981), with modification described by Chang and Lotti (Proc. Natl. Acad. Sci. USA, 83, 4923-4924, 1986).

Male Hartley guinea pigs (300-500 g) are sacrificed by decapitation, and the brains are removed and placed in ice-cold 50 mM Tris HCl (Trizma-7.4) [pH 7.4 at 25°C]. The cerebral cortex is dissected and used as a receptor source and each gram of fresh guinea pig brain tissue is homogenized in 10 ml of Tris/Trizma buffer with a Brinkmann polytron PT-10. The homogenates are centrifuged at 42,000g for 15 minutes, then the resulting pellets are resuspended in 200 volumes of binding assay buffer (10 mM N-2-hydroxyethylpiperazine-N'-2-ethanesulfonic acid (HEPES), 5 mM $MgCl_2$, 1 mM ethylene glycol-bis-(β-aminoethylether)-N,N'-tetraacetic acid (EGTA), 0.4% BSA (bovine serum albumin) and 0.25 mg/ml bacitracin, pH 6.5).

The remainder of the binding assay method is as described for the pancreas method, except that the reaction mixtures are incubated at 25°C for 2 hours before centrifugation.

Isolated guinea pig gall bladder method

The two halves of the gall bladders, free of adjacent tissue, of male Hartley guinea pigs (400-600g), which have been sacrificed by decapitation, are suspended under 1g tension along the axis of the bile duct in 5 ml organ bath, containing a Kreb's bicarbonate solution of 118 mM NaCl, 4.75 mM KCl, 2.54 mM $CaCl_2$, 1.19 mM $KH_2PO_4$, 1.2 mM $MgSO_4$, 25 mM $NaHCO_3$ and 11 mM dextrose, which is maintained at 32°C and bubbled with a mixture of 95% $O_2$ and 5% $CO_2$. The tissues are washed every 10 minutes for one hour to obtain equilibrium prior to the beginning of the study and the isometric contractions of the strips are recorded using Statham (60g:0.12 mm) strain gauges and a Hewlett-Packard 77588 recorder.

CCK-8 is added cumulatively to the baths and $EC_{50}$'s are determined using regression analysis. After washout (every 10 minutes for one hour), the compound to be tested is added at least 5 minutes before the addition of CCK-8 and the $EC_{50}$ of CCK-8 in the presence of compound to be tested is similarly determined.

A shift to the right of the CCK dose response curve without reduction of the maximal centractile response,

indicates competitive antagonism of CCK from this method.

Isolated longitudinal muscle of guinea pig ileum

Longitudinal muscle strips with attached nerve plexus are prepared as described in Brit. J. Pharmac. 23: ; 356-363, 1964; J. Physiol. 194: 13-33, 1969. Male Hartley guinea pigs are decapitated and the ileum removed (10 cm of the terminal ileum is discarded and the adjacent 20 cm piece used) with a 10 cm piece of the ileum being stretched on a glass pipette. Using a cotton applicator to stroke tangentially away from the mesentery attachment at one end, the longitudinal muscle is separated from the underlying circular muscle and the longitudinal muscle is tied to a thread and by gently pulling, stripped away from the entire muscle. A piece of approximately 2 cm is suspended in 5 ml organ bath containing Krebs solution and bubbled with 95% $O_2$ and 5% $CO_2$ at 37°C under 0.5 g tension. CCK-8 is added cumulatively to the baths and $EC_{50}$ values in the presence and absence of compounds to be tested are determined, as described in the gall bladder protocol above.

Gastrin Receptor Binding in Guinea Pig Gastric Glands

Guinea pig gastric mucosal glands are prepared by the procedure of Berglingh and Obrink, Acta Physiol. Scand. 96: 150 (1976), with a slight modification according to Praissman et al. C. J. Receptor Res. 3: (1983). Gastric mucosa from male Hartley guinea pigs ( 300-500 g body weight) are washed thoroughly and minced with fine scissors in standard buffer consisting of the following: 130 mM NaCl, 12 mM $NaHCO_3$, 3 mM $NaH_2PO_4$, 3 mM $Na_2HPO_4$, 3 mM $K_2HPO_4$, 2 mM $MgSO_4$, 1 mM $CaCl_2$, 5 mM glucose, 4 mM L-glutamine and 25 mM HEPES at pH 7.4. The minced tissues are washed and incubated in a 37°C shaker bath for 40 minutes, with the buffer containing 0.1% collagenase and 0.1% BSA, and bubbled with 95% $O_2$ and 5% $CO_2$. The tissues are passed twice through a 5 ml glass syringe to liberate the gastric glands, and then filtered through 200 mesh nylon. The filtered glands are centrifuged at 270 g for 5 minutes and washed twice by resuspension and centrifugation.

The washed guinea pig gastric glands are resuspended in 25 ml of standard buffer containing 0.25 mg/ml of bacitracin. For binding studies, 10 µl of buffer (for total binding) or gastrin (1 µM final concentration, for nonspecific binding) or test compound and 10 µl of $^{125}$I-gastrin (NEN, 2200 Ci/mmole, 25 pM final) or $^{3}$H-pentagastrin (NEN 22 Ci/mmole, 1 nM final) are added to 220 µl of gastric glands in triplicate tubes which are aerated with 95% $O_2$ and 5% $CO_2$ and capped. The reaction mixtures, after incubation at 25°C for 30 minutes, are filtered under reduced pressure on glass G/F B filters (Whatman) and immediately washed with 4 × 4 ml of standard buffer containing 0.1% BSA. The radioactivity on the filters is measured using a Beckman gamma 5500 for $^{125}$I-gastrin or liquid scintillation counting for $^{3}$H-pentagastrin.

The ability of the heterocycle-fused benzazepines of the instant invention to antagonize CCK and gastrin makes these compounds useful as pharmaceutical agents for mammals, especially for humans, for the treatment and prevention of disorders wherein CCK and/or gastrin may be involved. Examples of such disease states include gastrointestinal disorders, especially such as irritable bowel syndrome or ulcers, excess pancreatic or gastric secretion, acute pancreatis, or motility disorders; central nervous system disorders, caused by CCK interactions with dopamine, such as neuroleptic disorders, tardive dyskinesia, Parkinson's disease, psychosis or Gilles de la Tourette Syndrome; disorders of appetite regulatory systems; Zollinger-Ellison syndrome, antral G cell hyperplasia, or pain (potentiation of opiate analgesia); as well as certain tumors of the lower esophagus, stomach, intestines and colon.

The compounds of the instant invention or pharmaceutically-acceptable salts thereof, may be administered to a human subject either alone or, preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intraperitoneal, subcutaneous and topical administration.

For oral use of an antagonist of CCK, according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

When a compound according to the instant invention, or a salt thereof, is used as an antagonist of CCK or gastrin in a human subject, the daily dosage will normally be determined by the prescribing physician with the dosage generally varying according to the age, weight, and response of the individual patient, as well as the severity of the patient's symptoms. However, in most instances, an effective daily dosage will be in the range of from about 0.05 mg/kg to about 50 mg/kg of body weight, and preferably, of from 0.5 mg/kg to about 20 mg/kg of body weight, administered in single or divided doses. In some cases, however, it may be necessary to use dosages outside these limits.

In the treatment of irritable bowel syndrome, for instance, 1 to 10 mg/kg of a CCK antagonist might be

administered orally (p.o.), divided into two doses per day (b.i.d.). In treating delayed gastric emptying, the dosage range would probably be the same, although the drug might be administered either intravenously (I.V.) or orally, with the I.V. dose probably tending to be slightly lower due to better availability. Acute pancreatitis might be treated preferentially in an I.V. form, whereas spasm and/or reflex esophageal, chronic pancreatitis, past vagatomy diarrhea, or treatment of anorexia or of pain associated with biliary dyskinesia might indicate p.o. form administration.

In the use of a gastrin antagonist as a tumor palliative for gastrointestinal neoplasms with gastrin receptors, as a modulator of central nervous system activity treatment of Zollinger-Ellison syndrome, or in the treatment of peptic ulcer disease, a dosage of .1 to 10 mg/kg administered one-to-four times daily might be indicated.

Because these compounds antagonize the function of CCK in animals, they may also be used as feed additives to increase the food intake of animals in daily dosage of approximately 0.05 to 50 mg/kg of body weight.

The invention is further defined by reference to the following examples which are intended to be illustrative and not limiting.

EXAMPLE 1

1,5-Dihydro-2-methyl-7-phenyl-as-triazino[4,3-a][1,4]-benzodiazepine (20, $X^1=H$, $R^1=CH_3$, $R^2=Ph$, $R^3=H$)

This compound is prepared according to the method of Moffet et al., J. Heterocyclic Chem., 14, 1231-1244 (1977).

EXAMPLE 2

2-Methyl-5-oximino-7-phenyl-as-triazino]4,3-a][1,4]-benzodiazepine (38, $X^1=H$, $R^2=Ph$, $W=CH_2$, $Y=C\text{-}CH_3$, $Z=N$)

To a suspension of potassium tert-butoxide (24.9 g, 222 mmole) in 600 ml of dry tetrahydrofuran is added 200 ml of dry tert-butylalcohol at -20°C under nitrogen. To this solution is then added, via addition funnel, 1,5-dihydro-2-methyl-7-phenyl-as-triazino[4,3-a][1,4]-benzodiazepine (25 g) in 260 ml of tetrahydrofuran, and the resulting solution is stirred for 2 hours at -20°C and treated with 17.4 ml (130 mmole) of isoamyl nitrite. The reaction mixture is warmed to 0°C over 15 minutes and quenched with the addition of 60 ml of cold water and 20 ml of glacial acetic acid. All solvents are removed under reduced pressure and the residue is partitioned between ethyl acetate (600 ml) and brine (100 ml), with the phases being separated and the organic extracts dried ($Na_2SO_4$) and concentrated. The resulting product is triturated with ether to give the title compound.

EXAMPLE 3

5(R,S)-Amino-1,5-dihydro-2-methyl-7-phenyl-as-triazino[4,3-a][1,4]-benzodiazepine (39, $X^1=H$, $R^2=Ph$, $W=CH_2$, $Y=C\text{-}CH_3$, $Z=N$, $n=0$)

A solution of 150 ml of methanol containing 5 g of 2-methyl-5-oximino-7-phenyl-as-triazino[4,3-a][1,4]-benzodiazepine is treated with a slurry of active Raney-Nickel catalyst[1] in ethanol (10 g). The resulting suspension is hydrogenated on a Parr apparatus at 60 psi and 23°C for 30 hours, with the catalyst being removed by filtration and the filtrate concentrated to afford the title compound.

EXAMPLE 4

5(R,S)-(2(S)-tert-Butoxycarbonylamino-3-phenylpropanoylamino)-1,5-dihydro-2-methyl-7-phenyl-as triazino[4,3-a][1,4]-benzodiazapine

Crude 5(R,S)-amino-1,5-dihydro-2-methyl-7-phenyl-as-triazino[4,3-a][1,4]-benzodiazepine (1.37 g), Boc-L-phenylalanine (1.37 g, 5.17 mmole), 1-hydroxybenzotriazole (HBT) (0.70 g, 5.17 mmole), and 1-ethyl-3-(3-dimethylaminopropyl)carbodiimidehydrochloride (EDC) (0.99 g, 5.17 mmole) are combined in DMF (30 ml) and stirred at room temperature, with the pH of the mixture being adjusted to 8.5 with triethylamine. After 1/2 hour, the DMF is removed in vacuo and the residue is partitioned between ethyl acetate and 10% citric acid (10 ml). The layers are separated and the organic phase is washed with sodium bicarbonate solution. The combined organic layers are washed with brine, dried over $Na_2SO_4$, filtered, and evaporated to dryness in vacuo. The residue is chromatographed on silica gel and the combined product fractions evaporated to dryness in vacuo to give the title compound as a mixture of diastereomers.

EXAMPLE 5

5(R and S)-(2(S)-Amino-3-phenylpropanoylamino)-1,5-dihydro-2-methyl-7-phenyl-as-triazino[4,3-a][1,4]-benzodiazepine

5(R,S)-(2(S)-tert-Butoxycarbonylamino-3-phenylpropanoylamino)-1,5-dihydro-2-methyl-7-phenyl-as-triazino[4,3-a][1,4]-benzodiazepine (1.8 gm) is dissolved in EtOAc (25 ml), cooled to 0°C, and the solution saturated with HCl (g) over a 10 minute period. After stirring an additional 10 minutes, the solvent is removed in

[1] Raney-Nickel catalyst is prepared according to Fieser & Fieser, Reagents for Organic Synthesis, Vol. I, John Wiley & Sons, Inc., New York 1967, p. 729.

vacuo. The residue is dissolved in $H_2O$, basified with saturated $Na_2CO_3$ (aqueous) and extracted with EtOAc (3 ×). The organic layers are combined, washed with brine, dried over $Na_2SO_4$, filtered and rotoevaporated in vacuo to give a foam. Flash chromatography on silica gel separates the 1/1 pair of diastereomers into an upper and lower component. The fractions containing the individual components are concentrated to dryness to give the separated diastereomers.

EXAMPLE 6

5(R)- and 5(S)-Amino-1,5-dihydro-2-methyl-7-phenyl-as-triazino[4,3-a][1,4]-benzodiazepine

5(S)-(2(S)-Amino-3-phenylpropanoylamino)-1,5-dihydro-2-methyl-7-phenyl-as-triazino[4,3-a][1,4]- benzodiazepine (1.15 g) is combined with phenylisothiocyanate (395 mg, 2.93 mmole) in $CH_2Cl_2$ (20 ml) and the mixture concentrated on a steam bath. The resulting oil is twice diluted with $CH_2Cl_2$ (20 ml) and both times reconcentrated on the steam bath. The oil is evaporated in vacuo, with that product being treated with TFA (15 ml) and warmed for 18 minutes in an oil bath thermostatted at 52°. The TFA is removed in vacuo, and the residue is treated twice with $CH_2Cl_2$ and with $Et_2O$. The product is evaporated in vacuo after each treatment, and the resulting oil is chromatographed on silica gel. The product fractions are evaporated in vacuo, and the residue is dissolved in $CH_2Cl_2$, washed with a small volume of 5% NaOH, dried over $Na_2SO_4$, filtered, and evaporated to give the 5(S) isomer of the title structure.

5(R)-(2(S)-Amino-3-phenylpropanoylamino)-1,5-dihydro-2-methyl-7-phenyl-as-triazino[4,3-a][1,4]-benzodiazepine is prepared by the same procedure from the appropriate starting material to the 5(R) enantiomer of the title compound.

EXAMPLE 7

5(S)-1,5-Dihydro-5-(2-indolecarbonylamino)-2-methyl-7-phenyl-as-triazino[4,3-a][1,4]-benzodiazepine (43, $X^1$=H, $R^2$=Ph, $R^3$=NHCO-2-indole, W=$CH_2$, Y=C-$CH_3$, Z=N)

5(S)-5-Amino-1,5-dihydro-2-methyl-7-phenyl-as-triazino[4,3-a][1,4]]-benzodiazepine (595 mg) is dissolved in $CH_2Cl_2$ (15 ml) and treated with 2-indolecarbonyl chloride (403 mg, 2.24 mmole) followed by triethylamine (227 mg, 2.24 mmole). The mixture is stirred at room temperature for 30 minutes and concentrated in vacuo. The residue is chromatographed on silica gel and the combined product fractions evaporated to dryness in vacuo to give the title compound.

EXAMPLE 8

5(R)-1,5-Dihydro-5-(3-methoxyphenylaminocarbonylamino)-2-methyl-7-phenyl-as-triazino[4,3-a][1,4]benzodiazepine (43 $X^1$=H, $R^2$=Ph,

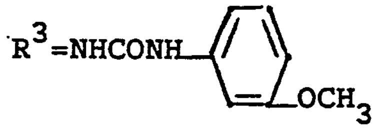,

$W = CH_2$, $Y = CH_3$, $Z = N$)

To a solution of 85 mg of 5(R)-amino-1,5-dihydro-2-methyl-7-phenyl-as-triazino[4,3-a][1,4]-benzodiazepine in 8 ml of dry tetrahydrofuran is added 3-methoxyphenylisocyanate (40 µl, 0.315 mmol) at room temperature. Stirring is continued for 8 hours more and the reaction mixture is filtered, with the collected solids being washed with hot methanol and dried in vacuo to give the title product.

EXAMPLE 9

3,5-Dihydro-3-methyl-7-phenyl-as-triazino[4,3-a][1,4]-benzodiazepin-2-(1H) one (17, $X^1 = H$, $R^1 = CH_3$, $R^2 =$ phenyl, $R^3 = H$)

This compound is prepared according to the method of Moffet, et al., J. Heterocyclic Chem., 14, 1231-1244 (1977).

EXAMPLE 10

3-Methyl-5-oximino-7-phenyl-as-triazino-[4,3-a][1,4]-benzodiazepin-2(1H)one (38, $X^1 = H$, $R^2 = Ph$, $W = CH_2$, $Y = \overset{O}{\overset{\|}{C}}$, $Z = N\text{-}CH_3$)

This compound is prepared according to the method of Example 2.

EXAMPLE 11

5-Amino-3,5-dihydro-3-methyl-7-phenyl-as-triazino[4,3-a][1,4]-benzodiazepin-2-(1H)one (39, $X^1 = H$, $R^2 = Ph$, $W = CH_2$, $Y = \overset{O}{\overset{\|}{C}}$, $Z = N\text{-}CH_3$, $n = 0$)

This compound is prepared according to the method of Example 3 and resolved according to the methods of Examples 4 through 6.

EXAMPLE 12

5(S)-5-(4-Chlorophenylcarbonylamino)-3,5-dihydro-3-methyl-7-phenyl-as-triazino[4,3-a][1,4]-benzodiazepin-2(1H)one (43, $X^1$=H, $R^2$=Ph,

-

$R^3$= 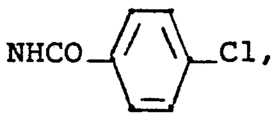

W=$CH_2$, Y= $\overset{O}{\overset{\|}{C}}$, Z=N-$CH_3$)

This compound is prepared according to the method of Example 17.

EXAMPLE 13

5(R)-3,5-Dihydro-3-methyl-5-(3-methylphenylaminocarbonylamino)-7-phenyl-as-triazino[4,3-a][1,4]benzodiazepin-2(1H)one (42, $X^1$=H, $R^2$=Ph,

-

$R^3$= 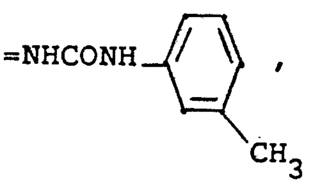

W=$CH_2$, Y= $\overset{O}{\overset{\|}{C}}$, Z=N-$CH_3$)

This compound is prepared according to the method of Example 8.

EXAMPLE 14

1-Methyl-7-phenyl-5H-pyrimido[4,3-a][1,4]-benzodiazepin-3-one (5, $X^1$=H, $R^2$=Ph, $R^3$=H)

This compound is prepared according to the methods of Kuwada et al., D.O.S. 2251291 (10 May 1973); Chem. Abstr., 79, 32117-(1973).

EXAMPLE 15

1-Methyl-5-oximino-7-phenyl-pyrimido [4,3-a][1,4]-benzodiazepin-3-one (38, $X^1$=H, $R^2$=Ph, W=C-$CH_3$, Y=CH,

Z= $\overset{O}{\overset{\|}{C}}$ )

This compound is prepared according to the methods of Example 2.

EXAMPLE 16

5-Amino-1-methyl-7-phenyl-5H-pyrimido [4,3-a][1,4]-benzodiazepin-3-one (39, $X^1$=H, $R^2$=Ph, W=C-$CH_3$, Y=CH,

Z= $\overset{O}{\overset{\|}{C}}$ , n=0

This compound is prepared according to the methods of Example 3 and resolved according to the methods of Examples 4 through 6.

EXAMPLE 17

5-(S)-5-(2-Indolecarbonylamino)-1-methyl-7-phenyl-5H-pyrimido-[4,3-a][1,4]-benzodiazepin-3-one (43, $X^1$=H, $R^2$=Ph, $R^3$=NHCO-2-indole, W=C-$CH_3$, Y=CH,

Z= $\overset{O}{\overset{\|}{C}}$ )

This compound is prepared according to the method of Example 7.

EXAMPLE 18

5(R)-5-(3-Chlorophenylaminocarbonylamino)-1-methyl-7-phenyl-5H-pyrimido-[4,3-a][1,4]-benzodiazepin-3-one (42, $X^1$=H, $R^2$=Ph,

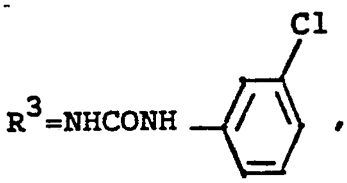

W=C-$CH_3$, Y=CH,

$Z=\overset{O}{\overset{\|}{C}}$ )

This compound is prepared according to the method of Example 8.

EXAMPLE 19

2-Methyl-7-phenyl-5H-pyrimido[4,3-a][1,4]-benzodiazepin-1-one (11, $X^1$=H, $R^2$=Ph, $R^3$=H)

This compound is prepared according to the methods of Szmuszkovicz, D.O.S. 2400449 (18 July 1974); Chem. Abstr., 81, 1055901+ (1974).

EXAMPLE 20

2-Methyl-5-oximino-7-phenylpyrimido[4,3-a][1,4]-benzodiazepin-1-one (38, $X^1$=H, $R^2$=Ph,

$W=\overset{O}{\overset{\|}{C}}$, Y=C-$CH_3$, Z=CH)

This compound is prepared according to the method of Example 2.

EXAMPLE 21

5-Amino-2-methyl-7-phenyl-5H-pyrimido[4,3-a][1,4]-benzodiazepin-1-one (39, $X^1$=H, $R^2$=Ph,

$W=\overset{O}{\overset{\|}{C}}$, Y=C-$CH_3$, Z=CH, n=0)

This compound is prepared according to the method of Example 3 and resolved according to the methods of Examples 4 through 6.

EXAMPLE 22

5(R)-5-(3-Methoxyphenylaminocarbonylamino)-2-methyl-7-phenyl-5H-pyrimido[4,3-a][1,4]-benzodiazepin-1-one (42, $X^1$=H, $R^2$=Ph,

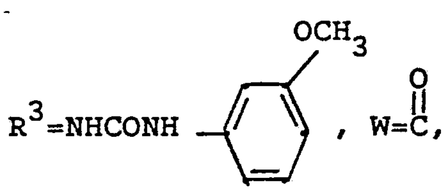

Y=C-$CH_3$, Z=CH)

This compound is prepared according to the method of Example 8.

EXAMPLE 23

5-(S)-5-(2-Indolecarbonylamino)-2-methyl-7-phenyl-5H-pyrimido[4,3-a][1,4]-benzodiazepin-1-one (43, $X^1$=H, $R^2$=Ph, $R^3$=NHCO-2-indole,

$W=\overset{O}{\overset{\|}{C}}$, Y=C-$CH_3$, Z=CH)

This compound is prepared according to the method of Example 7.

EXAMPLE 24

2-Methyl-7-phenyl-s-triazino-[4,3-a][1,4]-benzodiazepine-1,3-dione (14, $X^1=H$, $R^1=CH_3$, $R^2=Ph$, $R^3=H$)

This compound is prepared according to the methods of Moffet and Rudzik, J. Med. Chem., 16, 1256 (1973).

EXAMPLE 25

2-Methyl-5-oximino-7-phenyl-s-triazino-[4,3-a][1,4]-benzodiazepin-1,3-dione (38, $X^1=H$, $R^2=Ph$, $W=\overset{O}{\overset{\|}{C}}$, $Y=N\text{-}CH_3$, $Z=\overset{O}{\overset{\|}{C}}$

This compound is prepared according to the method of Example 2.

EXAMPLE 26

5-Amino-2-methyl-7-phenyl-s-triazino[4,3-a][1,4]-benzodiazepine-1,3,dione (39, $X^1=H$, $R^2=Ph$, $W=\overset{O}{\overset{\|}{C}}$, $Y=N\text{-}CH_3$, $Z=\overset{O}{\overset{\|}{C}}$, n=0).

This compound is prepared according to the method of Example 3 and resolved according to the methods of Examples 4 through 6.

EXAMPLE 27

5(S)-5-(2-Indolecarbonylamino)-2-methyl-7-phenyl-s-triazino[4,3-a][1,4]-benzodiazepine-1,3-dione (43, $X^1=H$, $R^2=Ph$, $R^3$=NHCO-2-indole, $W=\overset{O}{\overset{\|}{C}}$, $Y=N\text{-}CH_3$, $Z=\overset{O}{\overset{\|}{C}}$)

This compound is prepared according to the method of Example 7.

EXAMPLE 28

5(R)-5-(3-Methoxyphenylaminocarbonylamino)-2-methyl-7-phenyl-s-triazino-[4,3-a][1,4]-benzodiazepine-1,3-dione (42, $X^1=H$, $R^2=Ph$,

$R^3$=NHCONH 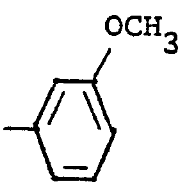 , $W=\overset{O}{\overset{\|}{C}}$,

$Y=N\text{-}CH3$, $Z=\overset{O}{\overset{\|}{C}}$ )

This compound is prepared according to the method of Example 8.

**Claims**

1. A compound of the formula:

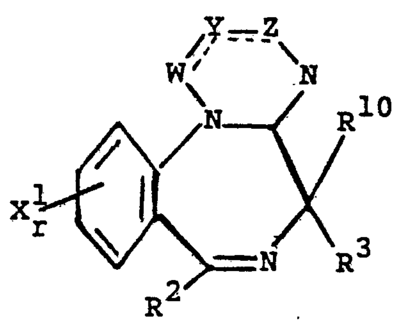

(I)

$R^1$ is H, $C_1$-$C_4$-alkyl, cyclo-$C_3$-$C_7$-alkyl,

$(CH_2)_n\overset{\overset{O}{\|}}{C}-R^9$, or $(CH_2)_mNR^4R^5$;

$R^2$ is H; $C_1$-$C_4$-alkyl; mono- or disubstituted or unsubstituted phenyl, where the substitutent(s) is/are independently selected from the group consisting of halo, $C_1$-$C_4$-alkyl, $C_1$-$C_4$-alkoxy, $C_1$-$C_4$-alkylthio, carboxyl, carboxy-$C_1$-$C_4$-alkyl, nitro, $-CH_3$, $O\overset{\overset{O}{\|}}{C}-R^4$ and hydroxy; -2, -3 or 4-pyridyl; or $-(CH_2)_mCOOR^6$;

$R^3$ is $-(CH_2)_nR^7$, $-(CH_2)_n\overset{\overset{OH}{|}}{C}HR^7$, $-(CH_2)_n\overset{\overset{O}{\|}}{C}R^7$,

$-(CH_2)_nNR^{18}(CH_2)_qR^7$, $-(CH_2)_nX^9\overset{\overset{O}{\|}}{C}(CH_2)_qR^7$,

$-(CH_2)_nX^9\overset{\overset{O}{\|}}{C}\underset{\underset{NHCOOR^{14}}{|}}{C}HCH_2R^7$,

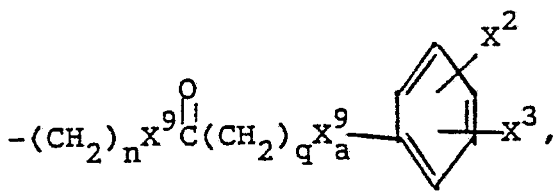

$-(CH_2)_n-X^9-\overset{\overset{O}{\|}}{C}-X^9_a-(CH_2)_q-R^7$, or

$-(CH_2)_n-X^9-\overset{\overset{O}{\|}}{C}-\overset{\overset{NH_2}{|}}{C}H-CH_2R^7$;

$R^4$ and $R^5$ are independently H, $C_1$-$C_4$-alkyl, or cyclo-$C_3$-$C_7$-alkyl or are connected to form a hetero ring of the structure $-\overset{\frown}{N(CH_2)_k}$, wherein k is 2 to 6;

$R^6$ is H, $C_1$-$C_4$-alkyl, cyclo-$C_3$-$C_7$-alkyl, unsubstituted or mono- or disubstituted phenyl, where the substituent(s) is/are independently selected from the group consisting of halo, $C_1$-$C_4$-alkyl, $C_1$-$C_4$-alkoxy, nitro, and $CF_3$, or unsubstituted or mono- or disubstituted phenyl-$C_1$-$C_4$-alkyl, where the substituent(s) is/are independently selected from the group consisting of halo, $C_1$-$C_4$-alkyl, $C_1$-$C_4$-alkoxy, nitro, and $CF_3$;

$R^7$ is $\alpha$- or $\beta$-naphthyl, unsubstituted or mono- or disubstituted phenyl, where the substituent(s) is/are independently selected from the group consisting of halo, $-NO_2$, -OH, $-NR^4R^5$, $C_1$-$C_4$-alkyl, cyano, phenyl, trifluoromethyl, acetylamino, acetyloxy, $C_1$-$C_4$-alkylthio, $SCF_3$, $C{\equiv}CH$, $CH_2SCF_3$, S-phenyl, and $C_1$-$C_4$-alkoxy,

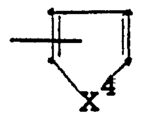, 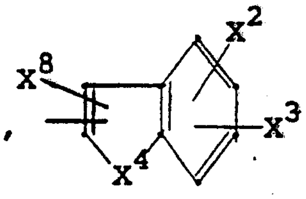, 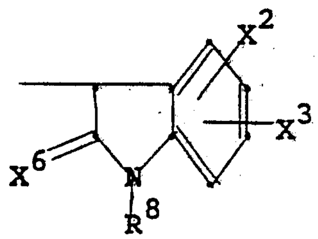

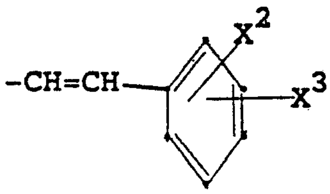, or 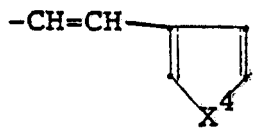,

$R^8$ is H, $C_1$-$C_4$-alkyl, cyclo-$C_3$-$C_7$-alkyl, $-(CH_2)_n$-cyclo-$C_3$-$C_7$-alkyl, $-\overset{O}{\overset{\|}{C}}-C_1$-$C_4$-alkyl,

or $-CO\underset{\underset{CH_2R^{12}}{|}}{C}HNHCOOR^{11}$;

$R^9$ is OH, $OR^{11}$

or 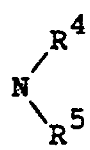;

$R^{10}$ is H, -OH, or $-CH_3$;
$R^{11}$ and $R^{12}$ are independently $C_1$-$C_4$-alkyl or cyclo-$C_3$-$C_7$-alkyl;
$R^{14}$ is $C_1$-$C_4$-alkyl or phenyl-$C_1$-$C_4$-alkyl;
$R^{18}$ is H, $C_1$-$C_4$-alkyl, formyl, acetyl, propionyl or butyryl;
m is 1 to 4;
n is 0 to 4;
q is 0 to 4;
r is 1 or 2;
$X^1$ is H, $-NO_2$, $CF_3$ CN, OH, $C_1$-$C_4$-alkyl, halo, $C_1$-$C_4$-alkylthio, $C_1$-$C_4$alkoxy, $-(CH_2)_nCOOR^6$, $-NR^4R^5$, or $O-\overset{O}{\overset{\|}{C}}-R^4$;
$X^2$ and $X^3$ are independently H, -OH, $-NO_2$, halo, $C_1$-$C_4$-alkylthio, $C_1$-$C_4$-alkyl, $C_1$-$C_4$-alkoxy or $-O-\overset{O}{\overset{\|}{C}}-R^4$;
$X^4$ is S, O, $CH_2$, or $NR^8$;
$X^6$ is O or HH;
$X^8$ is H or $C_1$-$C_4$-alkyl;
$X^9$ and $X^9_a$ are independently $NR^{18}$ or O;
W is $CR^1$, $CHR^{10}$, or $\overset{O}{\overset{\|}{C}}$;
Y is $N-R^1$, $C-R^1$, or $\overset{O}{\overset{\|}{C}}$;
Z is N, $NR^1$, $CR^1$, or $\overset{O}{\overset{\|}{C}}$;
--- is a saturated (single) or unsaturated (double bond);
or a pharmaceutically-acceptable salt thereof.

2. A compound according to Claim 1, wherein
$R^1$ is H or $C_1$-$C_4$-alkyl;
$R^2$ is unsubstituted of mono- or disubstituted phenyl, where the substitutent(s) is/are independently selected from the group consisting of halo, $C_1$-$C_4$-alkyl, $C_1$-$C_4$-alkoxy, nitro, and $-CF_3$;

$R^3$ is $-(CH_2)_nR^7$, $-(CH_2)_nX^9\overset{O}{\overset{\|}{C}}(CH_2)_qR^7$ or

$-(CH_2)_n-X^9-\overset{O}{\overset{\|}{C}}-X\quad-(CH_2)_q-R^7$;
$R^7$ is α- or β-naphthyl, unsubstituted or mono- or disubstituted phenyl, where the substituent(s) is/are independently selected from the group consisting of halo, nitro, $C_1$-$C_4$-alkyl, trifluoromethyl and $C_1$-$C_4$-alkoxy,

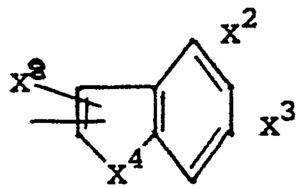 , or 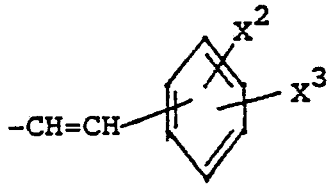 ;

$R^8$ is H, or $C_1$-$C_4$-alkyl;
n is 0 -to- 2;
q is 0 -to- 2;
r is 1 or 2;
$X^1$ is H, $-NO_2$, $C_1$-$C_4$-alkyl, halo, or $C_1$-$C_4$-alkoxy;
$X^2$ and $X^3$ are independently H, $-NO_2$, halo, $C_1$-$C_4$-alkyl or $C_1$-$C_4$-alkoxy;
$X^4$ is S, O, $CH_2$, or $NR^8$;
$X^8$ is H or $C_1$-$C_4$-alkyl;
$X^9$ and $X^9_a$ are independently NH or O;
or a pharmaceutically acceptable salt thereof.
A compound according to Claim 1, wherein
$R^1$ is H or methyl;
$R^2$ is phenyl or o-F-phenyl;

$R^3$ is NH -$R^7$ or NH NH-$R^7$;
$R^7$ is

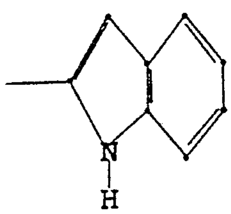 , 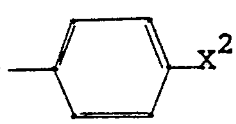 , 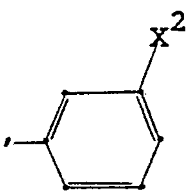 or 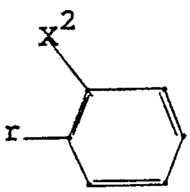 ;

$X^1$ is H;
$X^2$ is H, $-NO_2$, halo, methyl, or methoxy;
W is $CH_2$;
Y is C-$R^1$; and
Z is N.
4. A compound according to Claim 1, wherein
$R^1$ is H or methyl;
$R^2$ is phenyl or o-F-phenyl;

$R^3$ is NH $\overset{O}{\overset{\|}{C}}$-$R^7$ or NH $\overset{O}{\overset{\|}{C}}$ NH-$R^7$;
$R^7$ is

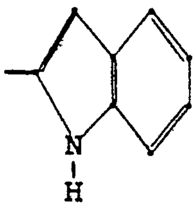 , 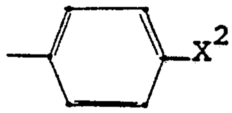 , 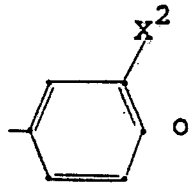 or 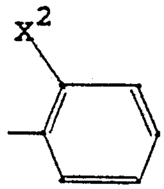 ;

$X^1$ is H;
$X^2$ is H, $-NO_2$, halo, methyl, or methoxy;
W is $CH_2$;
Y is $\overset{O}{\overset{\|}{C}}$; and
Z is $N-R^1$.

5. A compound according to Claim 1, wherein
$R^1$ is H or methyl;
$R^2$ is phenyl or o-F-phenyl;
$R^3$ is $NH\overset{O}{\overset{\|}{C}}-R^7$ or $NH\overset{O}{\overset{\|}{C}}NH-R^7$;
$R^7$ is

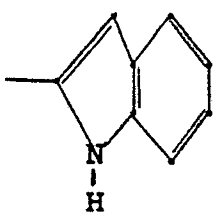 , 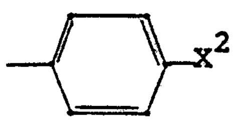 , 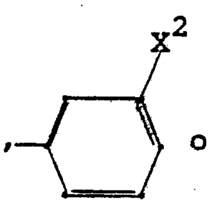 or 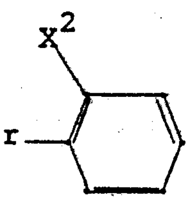 ;

$X^1$ is H;
$X^2$ is H, $-NO_2$, halo, methyl, or methoxy;
W is $C-R^1$;
Y is CH; and
Z is $\overset{O}{\overset{\|}{C}}$.

6. A compound according to Claim 1, wherein
$R^1$ is H or $CH_3$;
$R^2$ is phenyl or o-F-phenyl;
$R^3$ is $NH\overset{O}{\overset{\|}{C}}-R^7$ or $NH\overset{O}{\overset{\|}{C}}NH-R^7$;
$R^7$ is

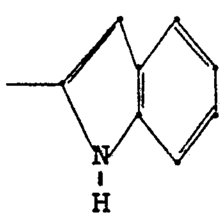 , 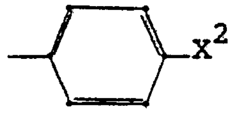 , 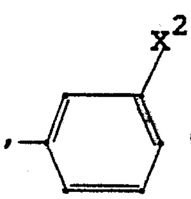 or 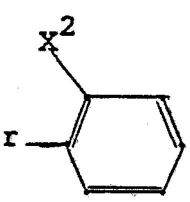 ;

$X^1$ is H;
$X^2$ is H, $-NO_2$, halo, methyl, or methoxy;
W is $\overset{O}{\overset{\|}{C}}$;
Y is $CR^1$; and
Z is CH.

7. A compound according to Claim 1, wherein
$R^1$ is H or $CH_3$;

$R^2$ is phenyl or o-F-phenyl;

$R^3$ is NH $\overset{O}{\overset{\|}{C}}$-$R^7$ or NH $\overset{O}{\overset{\|}{C}}$ NH-$R^7$;
$R^7$ is

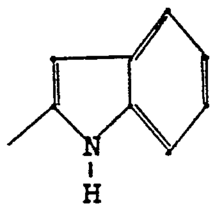 , 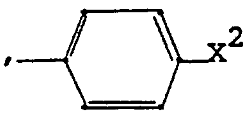 , 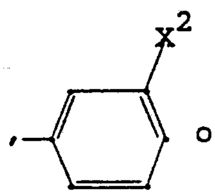 or 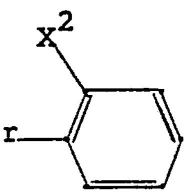 ;

$X^1$ is H;
$X^2$ is H, -$NO_2$, halo, methyl, or methoxy;

W is $\overset{O}{\overset{\|}{C}}$;
Y is N-$R^1$; and

Z is $\overset{O}{\overset{\|}{C}}$.

8. A compound according to Claim 1 which is:
5(S)1,5-dihydro-5-(2-indolecarbonylamino)-2-methyl-7-phenyl-as-triazino[4,2-a][1,4]benzodiazepine;
5(S)-5-(4-chlorophenylcarbonylamino)-3,5-dihydro-3-methyl-7-phenyl-as-triazino-[4,3-a][1,4]benzodiazepine-2(1H)one;
5(S)-5-(2-indolecarbonylamino)-1-methyl-7-phenyl-5H-pyrimido[4,3-a][1,4]-benzodiazepin-3-one; or
5(S)-5-(2-indolecarbonylamino)-2-methyl-7-phenyl-5H-pyrimido-[4,3-a][1,4]-benzodiazepin-1-one; or
5(S)-5-(2-indolecarbonylamino)-2-methyl-7-phenyl-s-triazino-[4,3-a][1,4]-benzodiazepin-1,3-dione.

9. A compound according to Claim 1 which is:
5(R)1,5-dihydro-5-(3-methoxyphenylaminocarbonylamino)-2-methyl-7-phenyl-as-triazino[4,3-a][1,4]benzodiazepine;
5(R)3,5-dihydro-3-methyl-5-(3-methylphenylaminocarbonylamino)-7-phenyl-as-triazino[4,3-a][1,4]-benzodiazepine-2(1H)one;
5(R)-5-(3-chlorophenylaminocarbonylamino)1-methyl-7-phenyl-5H-pyrimido-[4,3-a][1,4]-benzodiazepine-3-one;
5(R)-5-(3-methoxyphenylaminocarbonylamino)-2-methyl-7-phenyl-5H-pyrimido-[4,3-a][1,4]-benzodiazepine-1-one; or
5(R)-5-(3-methoxyphenylaminocarbonylamino)-2-methyl-7-phenyl-s-triazino-[4,3-a][1,4]-benzodiazepin-1,3-dione.

10. A composition comprising a therapeutically-effective amount for antagonism of the function of cholecystokinins or gastrin in mammals of one or more compounds according to Claim 1, or pharmaceutically-acceptable salts thereof, and a pharmaceutically-acceptable carrier.

11. A composition according to Claim 10. wherein the therapeutically-effective amount is about 0.5 to 50 mg/kg of body weight.

13. A composition according to Claim 10, wherein the mammals are humans.

14. The use of a compound as claimed in Claim 1 for the preparation of a medicament useful for preventing or treating disorders of the gastrointestinal, central nervous or appetite regulatory systems in mammals.

15. The use according to Claim 14, wherein the medicament contains a pharmaceutically-acceptable carrier or a pharmaceutically-acceptable carrier and an adjuvant.

16. The use of a compound according to Claim 1 for the preparation of a food additive useful for increasing food intake.

European Patent Office

# EUROPEAN SEARCH REPORT

Application number

EP 87311033.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 187 306 (K.H. MAYER et al.)<br>* Claims 1,15,16 * | 1,2,14, 15 | C 07 D 487/04<br>A 61 K 31/55//<br>(C 07 D 487/04,<br>C 07 D 257:00,<br>C 07 D 243:00)<br>(C 07 D 487/04,<br>C 07 D 253:00,<br>C 07 D 243:00)<br>(C 07 D 487/04,<br>C 07 D 251:00,<br>C 07 D 243:00)<br>(C 07 D 487/04,<br>C 07 D 243:00,<br>C 07 D 239:00) |
| A | EP - A1 - 0 189 803 (MERCK & CO. INC.)<br>* Claims 1,11-16 * | 1,2,10-15 | |
| A | EP - A2 - 0 169 392 (MERCK & CO. INC.)<br>* Claims 1,2,8-11; pages 5,12-14 * | 1,2,10-15 | |
| | | | TECHNICAL FIELDS SEARCHED (Int. Cl.4) |
| | | | C 07 D 487/00 |

The present search report has been drawn up for all claims

| Place of search | Date of completion of the search | Examiner |
|---|---|---|
| VIENNA | 23-03-1988 | PETROUSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82